(19) Europäisches Patentamt
European Patent Office
Office européen des brevets

(11) EP 4 414 409 B1

(12) **EUROPEAN PATENT SPECIFICATION**

(45) Date of publication and mention of the grant of the patent:
**17.09.2025 Bulletin 2025/38**

(21) Application number: **22878468.2**

(22) Date of filing: **03.10.2022**

(51) International Patent Classification (IPC):
***C08G 64/20*** (2006.01) ***C08J 11/20*** (2006.01)
***C08J 11/14*** (2006.01) ***C08G 64/06*** (2006.01)
***C08G 64/42*** (2006.01)

(52) Cooperative Patent Classification (CPC):
**C08G 64/42; C08G 64/06; C08J 11/14; C08J 11/20;**
Y02W 30/62

(86) International application number:
**PCT/JP2022/036961**

(87) International publication number:
**WO 2023/058599 (13.04.2023 Gazette 2023/15)**

(54) **DIHYDROXY COMPOUND MANUFACTURING METHOD AND RECYCLED RESIN MANUFACTURING METHOD**

VERFAHREN ZUR HERSTELLUNG EINER DIHYDROXYVERBINDUNG UND VERFAHREN ZUR HERSTELLUNG VON RECYCELTEM HARZ

PROCÉDÉ DE FABRICATION D'UN COMPOSÉ DIHYDROXY ET PROCÉDÉ DE FABRICATION DE RÉSINE RECYCLÉE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **05.10.2021 JP 2021164219**

(43) Date of publication of application:
**14.08.2024 Bulletin 2024/33**

(73) Proprietor: **MITSUBISHI GAS CHEMICAL COMPANY, INC.**
**Chiyoda-ku**
**Tokyo 100-8324 (JP)**

(72) Inventors:
- **KATO, Noriyuki**
  **Tokyo 100-8324 (JP)**
- **NISHIMORI, Katsushi**
  **Tokyo 125-8601 (JP)**
- **MOTEGI, Atsushi**
  **Tokyo 125-8601 (JP)**
- **ISHIHARA, Kentaro**
  **Tokyo 100-8324 (JP)**
- **MURATA SUZUKI, Shoko**
  **Tokyo 125-8601 (JP)**
- **TAKAMATSU, Kazutaka**
  **Tokyo 125-8601 (JP)**

(74) Representative: **Hoffmann Eitle**
**Patent- und Rechtsanwälte PartmbB**
**Arabellastraße 30**
**81925 München (DE)**

(56) References cited:
**WO-A1-2015/068628** **WO-A1-2016/052370**
**WO-A1-2016/052370** **WO-A1-2019/146507**
**WO-A1-2019/146507** **WO-A1-2021/200892**
**JP-A- 2003 171 324** **JP-A- 2015 110 555**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 414 409 B1

## Description

### Technical Field

The present invention relates to a dihydroxy compound manufacturing method, a recycled resin manufacturing method, and the like.

### Background Art

In recent years, there have been growing concerns about the deterioration of the natural environment and the increase in the amount of discharged wastes, and a movement to reuse and recycle plastic products has been further strengthen with an aim to realize a recycling-oriented society.

Synthetic resins such as polycarbonate resins, which are main constituent components of plastic products, have been widely used in various applications such as home electric appliances, electronic and electrical equipment, office automation equipment, optical media, automobile parts, and building members. Because a large amount of waste materials of the synthetic resins have been discharged in the manufacturing of the plastic products or after use of the plastic products, these waste materials have been reused.

In particular, when molding synthetic resins to manufacture plastic products, portions originating from mold passages, such as sprues, runners, and gates are removed to form the plastic products. Such synthetic resins that are unneeded for and removed from the plastic products, and other waste resins, such as defective moldings, are not discarded, and efforts have been made to recycle and reuse them in products.

For example, Patent Literature 1 describes an invention related to a recovery method for polycarbonate resin, the method including a step in which discarded optical disks and/or recovered optical disks having polycarbonate resin substrates are pulverized and the obtained pulverized product are chemically treated. In the recovery method, the chemically treated product obtained in the chemical treatment step is described to be subjected to a step of removing magnetic metal foreign substances by using a magnet and removing colored foreign substances by using an optical camera, and a step of removing resins containing metal foreign substances by using a metal detector for foreign substances.

### Citation List

#### Patent Literature

Patent Literature 1: JP 2011-131507 A

### Summary of Invention

#### Technical Problem

According to the recovery method described in Patent Document 1, metals, colored foreign substances, resins including metal foreign substances, and the like can be removed to recover synthetic resins. The recovery method described above is carried out based on the contained metal or colored appearance. However, plastic products are often manufactured by combining a plurality of types of synthetic resins, and thus organic impurities along with the desired synthetic resin are contained in the synthetic resins that are unneeded for and removed from plastic products, defective moldings, and the like. In this case, the presence or absence of metal content and the degree of coloring may be not significantly different from each other between the desired synthetic resin and the organic impurities. In this occasion, the desired synthetic resin cannot be recovered and recycled by the method described in Patent Document 1. Under such circumstances, a new method for recycling a waste resin composition is required.

Therefore, an object of the present invention is to provide a method for recycling a waste resin composition containing a synthetic resin and organic impurities.

#### Solution to Problem

The present invention has, for example, the following aspects.

[1] A method of manufacturing a dihydroxy compound from a waste resin composition, the method including:

a step (a1) in which an alkaline solution containing a first solvent, water, and a waste resin composition containing

a resin having at least two constitutional units selected from the group consisting of general formulae (1) to (4) set forth below:

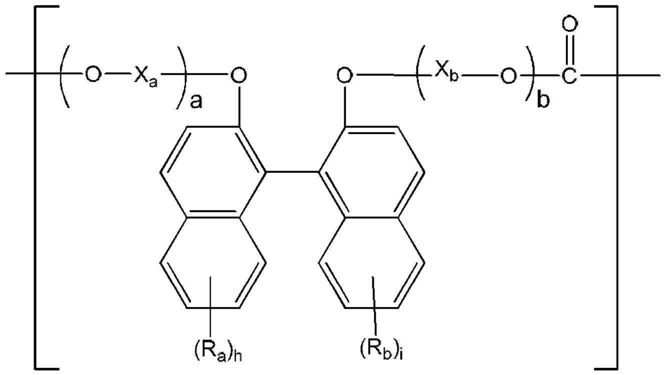

(1)

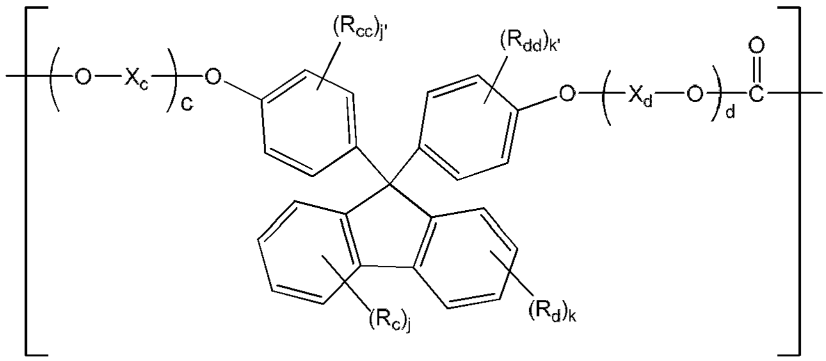

(2)

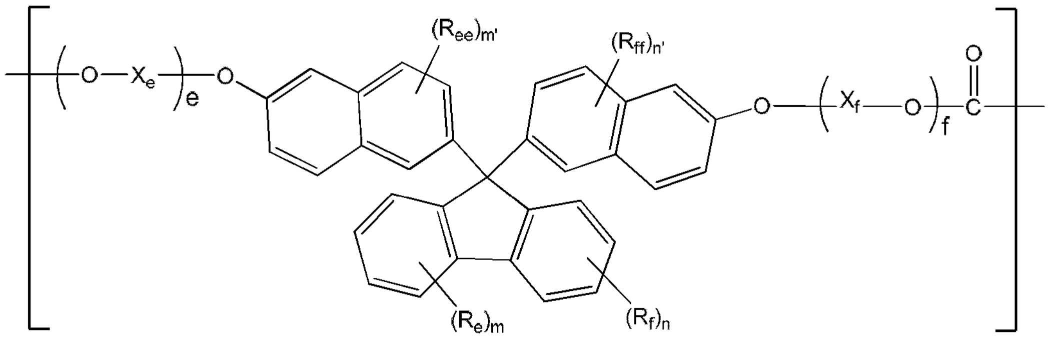

(3)

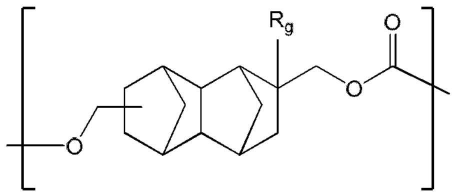

(4)

[wherein

$X_a$, $X_b$, $X_c$, $X_d$, $X_e$, and $X_f$ each independently represent an alkylene group having 1 to 4 carbon atoms,

$R_a$, $R_b$, $R_c$, $R_{cc}$, $R_d$, $R_{dd}$, $R_e$, $R_{ee}$, $R_f$, and $R_{ff}$ are each independently selected from a halogen atom, an alkyl group having 1 to 20 carbon atoms, an alkoxy group having 1 to 20 carbon atoms, a cycloalkyl group having 5 to 20 carbon atoms, a cycloalkoxy group having 5 to 20 carbon atoms, an aryl group having 6 to 20 carbon atoms, a heteroaryl group having 3 to 20 carbon atoms and containing one or more heterocyclic atoms selected from O, N, and S, an aryloxy group having 6 to 20 carbon atoms, and -C≡C-$R_i$,

$R_i$ represents an aryl group having 6 to 20 carbon atoms or a heteroaryl group having 3 to 20 carbon atoms and containing one or more heterocyclic atoms selected from O, N and S,

a, b, c, d, e, and f each independently represent an integer from 0 to 10,
h, i, j, j', k, k', m, m', n, and n' each independently represent an integer from 0 to 4, and
each $R_g$ independently represents a hydrogen atom or an alkyl group having 1 to 3 carbon atoms]
is treated to give a reaction solution containing a first solvent and a mixture of dihydroxy compounds containing a first dihydroxy compound selected from the group consisting of general formulae (1') to (4') set forth below:

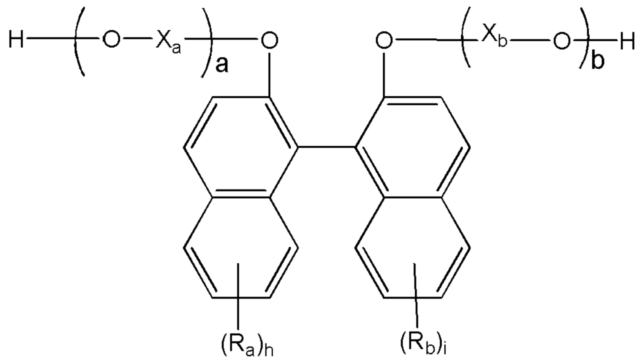

(1')

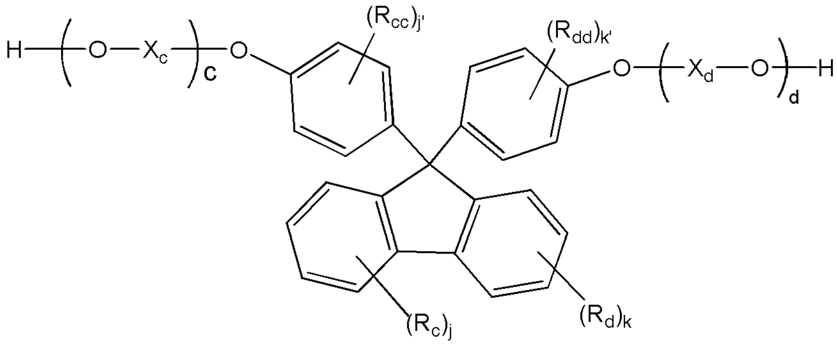

(2')

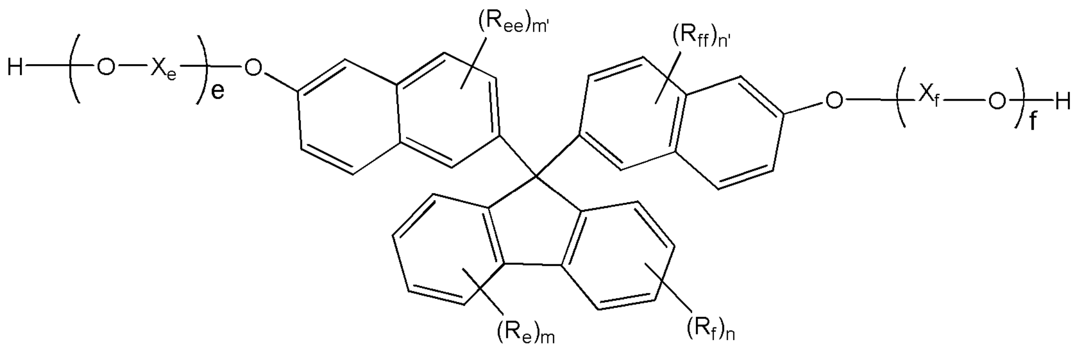

(3')

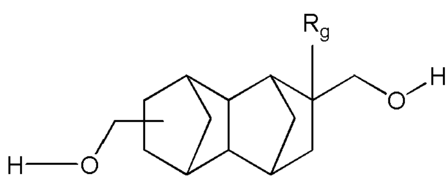

(4')

[wherein, each symbol represents the same as in the general formulae (1) to (4) set forth above]
and at least one other dihydroxy compound selected from the group consisting of the general formulae (1') to (4'); and
a step (b1) in which the first dihydroxy compound is crystallized from a crystallization solution obtained by adding a second solvent to the reaction solution,
wherein a difference between the solubility of the first dihydroxy compound in the second solvent at 25°C and the solubility of the other dihydroxy compound in the second solvent at 25°C (solubility of another dihydroxy compound minus solubility of the first dihydroxy compound) is greater than or equal to 0.1 g/(10 mL).

[2] The method according to [1], wherein the crystallization solution further contains a seed crystal.
[3] The method according to [1] or [2], wherein a content of the first solvent in the crystallization solution is from 0.1 to 10 g/g with respect to a total amount of the mixture of the dihydroxy compounds.
[4] The method according to any of [1] to [3], wherein a content of the second solvent in the crystallization solution is from 0.3 to 3 g/g with respect to a total amount of the mixture of the dihydroxy compounds.
[5] The method according to any of [1] to [4], wherein a total content of the first solvent and the second solvent in the crystallization solution is from 1 to 10 g/g with respect to a total amount of the mixture of the dihydroxy compounds.
[6] The method according to any of [1] to [5], wherein a ratio of a content (g/g) of the first solvent to a content (g/g) of the second solvent in the crystallization solution (first solvent/second solvent) is from 0.8 to 10.
[7] A method of manufacturing a dihydroxy compound from a waste resin composition, the method including:

a step (a2) in which an alkaline solution containing a first solvent, water, and a waste resin composition having at least two constitutional units selected from the group consisting of general formulae (1) to (4) set forth below:

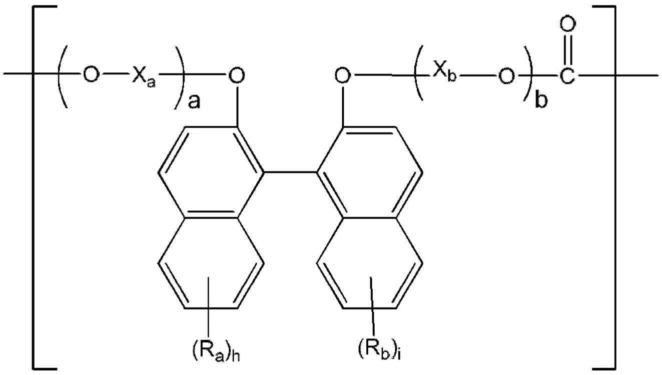

(1)

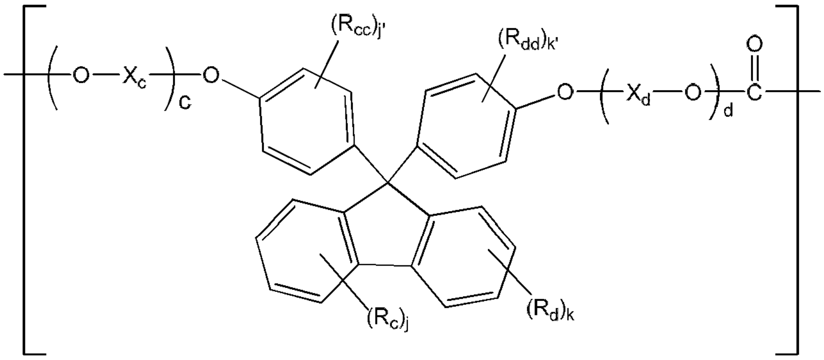

(2)

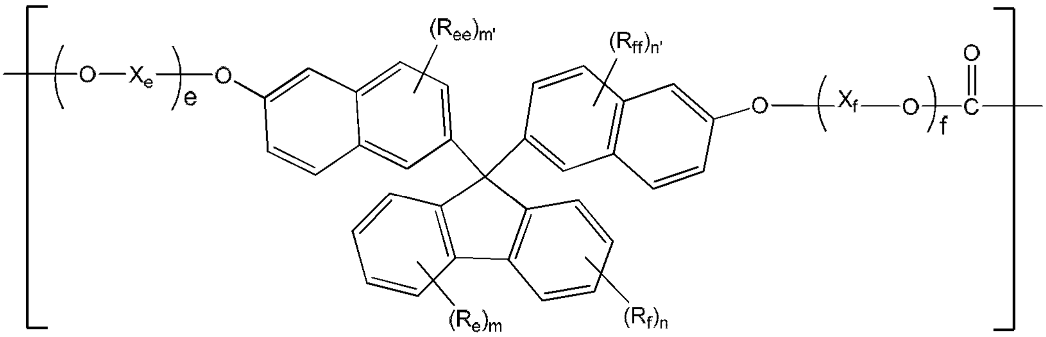

(3)

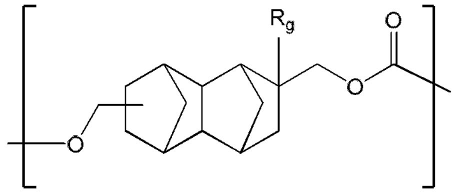

(4)

[wherein,

$X_a$, $X_b$, $X_c$, $X_d$, $X_e$, and $X_f$ each independently represent an alkylene group having 1 to 4 carbon atoms,

$R_a$, $R_b$, $R_c$, $R_{cc}$, $R_d$, $R_{dd}$, $R_e$, $R_{ee}$, $R_f$, and $R_{ff}$ are each independently selected from a halogen atom, an alkyl group having 1 to 20 carbon atoms, an alkoxy group having 1 to 20 carbon atoms, a cycloalkyl group having 5 to 20 carbon atoms, a cycloalkoxy group having 5 to 20 carbon atoms, an aryl group having 6 to 20 carbon atoms, a heteroaryl group having 3 to 20 carbon atoms and containing one or more heterocyclic atoms selected from O, N, and S, an aryloxy group having 6 to 20 carbon atoms, and $-C{\equiv}C-R_i$,

$R_i$ represents an aryl group having 6 to 20 carbon atoms or a heteroaryl group having 3 to 20 carbon atoms and containing one or more heterocyclic atoms selected from O, N and S,

a, b, c, d, e, and f each independently represent an integer from 0 to 10,

h, i, j, j', k, k', m, m', n, and n' each independently represent an integer from 0 to 4, and

each $R_g$ independently represents a hydrogen atom or an alkyl group having 1 to 3 carbon atoms]

is treated to give a reaction solution containing a first solution and a mixture of dihydroxy compounds containing a first dihydroxy compound selected from the group consisting of general formulae (1') to (4') set forth below:

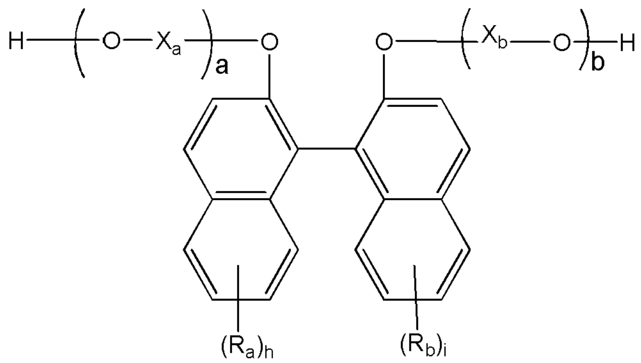

(1')

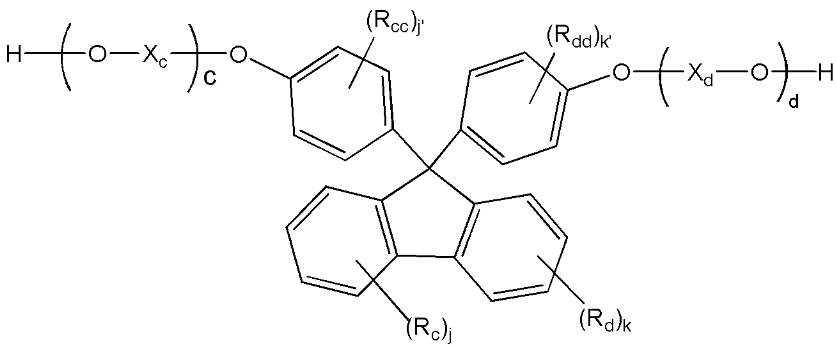

(2')

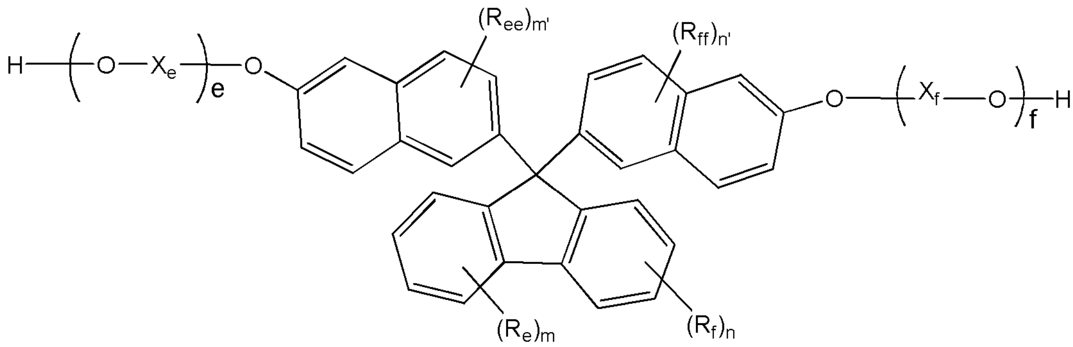

(3')

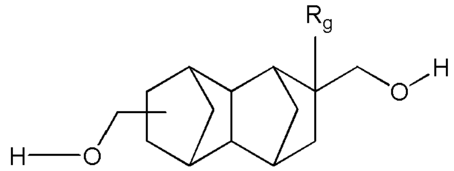

(4')

[wherein, each symbol represents the same as in the general formulae (1) to (4) set forth above]
and at least one other dihydroxy compound selected from the group consisting of the general formulae (1') to (4'); and
a step (b2) in which at least the first dihydroxy compound is crystallized from the reaction solution, wherein the step (b1) includes heating a reaction solution to 80°C or higher.

[8] The method according to any of [1] to [7], wherein the solution of the waste resin composition includes an impurity resin having at least one constitutional unit selected from the group consisting of general formulae (6) to (8) set forth below:

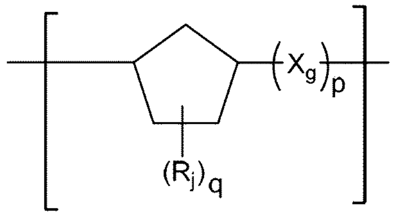

(6)

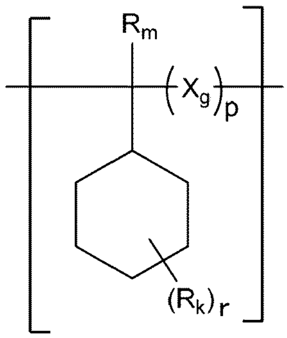

(7)

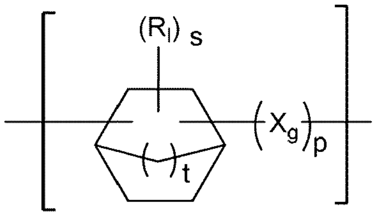

(8)

[wherein,

each $X_g$ independently represents an alkylene group having 1 to 10 carbon atoms,

$R_j$, $R_k$, and $R_l$ are each independently selected from a halogen atom, a substituted or unsubstituted alkyl group having 1 to 20 carbon atoms, a substituted or unsubstituted alkoxy group having 1 to 20 carbon atoms, a substituted or unsubstituted cycloalkyl group having 5 to 20 carbon atoms, a substituted or unsubstituted cycloalkoxy group having 5 to 20 carbon atoms, a substituted or unsubstituted aryl group having 6 to 20 carbon atoms, a substituted or unsubstituted heteroaryl group having 3 to 20 carbon atoms and containing one or more heterocyclic atoms selected from O, N, and S, a substituted or unsubstituted aryloxy group having 6 to 20 carbon atoms, and $-C \equiv C-R_i$,

$R_i$ represents a substituted or unsubstituted aryl group having 6 to 20 carbon atoms or heteroaryl group having 3 to 20 carbon atoms and containing one or more heterocyclic atoms selected from O, N, and S,

each p independently represents an integer of 0 or 1,

q, r, and s each independently represent an integer from 0 to 10,

t represents an integer from 1 to 3,

wherein when q is 2 or more and two $R_j$'s are present at adjacent carbon atoms, the two $R_j$'s may be bound together to form a ring structure,

when r is 2 or more and two $R_k$'s are present at adjacent carbon atoms, the two $R_k$'s may be bound together to form a ring structure,

when s is 2 or more and two Ri's are present at adjacent carbon atoms, the two $R_l$'s may be bound together to form a ring structure, and

$R_m$ represents a hydrogen atom or an alkyl group having 1 to 3 carbon atoms].

[9] A method of manufacturing a recycled resin, including polymerizing a dihydroxy compound manufactured by the method according to any of [1] to [8].

Advantageous Effects of Invention

According to the present invention, a waste resin composition containing a synthetic resin and organic impurities can be recycled.

Description of Embodiments

Hereinafter, the embodiments of the present invention will be described in detail.

<First embodiment: Dihydroxy compound manufacturing method>

According to the first embodiment of the present invention, there is provided a method of manufacturing a dihydroxy compound from a waste resin composition. **In** this context, the method includes step (a1) in which an alkaline solution containing a first solvent, water, and a waste resin composition containing a resin having at least two constitutional units selected from the group consisting of general formulae (1) to (4) set forth below is treated to give a reaction solution containing a first solvent and a mixture of dihydroxy compounds containing a first dihydroxy compound selected from the group consisting of general formulae (1') to (4') set forth below and at least one other dihydroxy compound selected from the group consisting of the general formulae (1') to (4'); and step (b1) in which the first dihydroxy compound is crystallized from a crystallization solution obtained by adding a second solvent to the reaction solution. In addition, the difference between the solubility of the first dihydroxy compound at 25°C in the second solvent and the solubility of the other dihydroxy compound at 25°C in the second solvent (solubility of the other dihydroxy compound minus solubility of the first dihydroxy compound) is greater than or equal to 0.1 g/(10 mL).

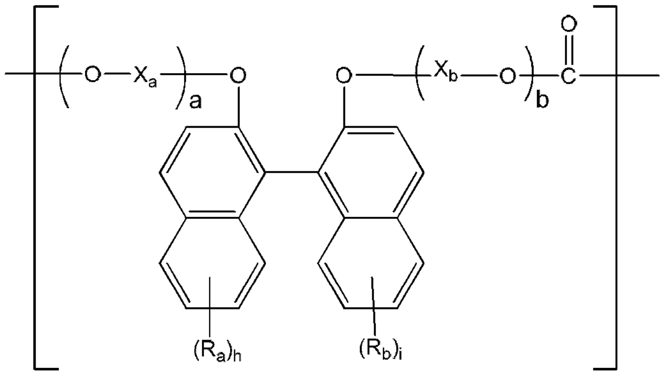

(1)

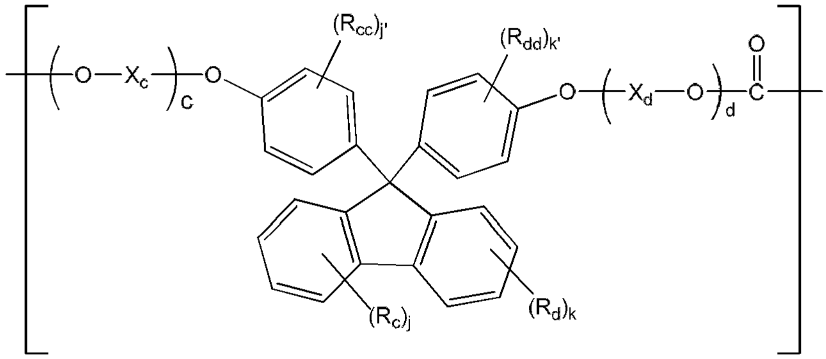

(2)

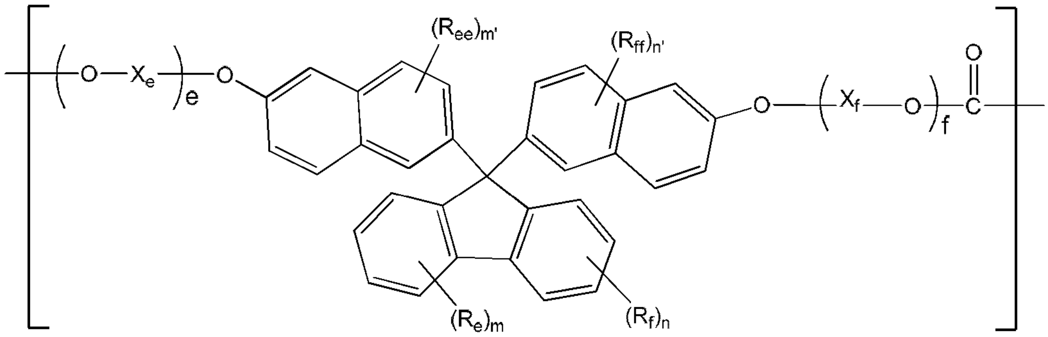

(3)

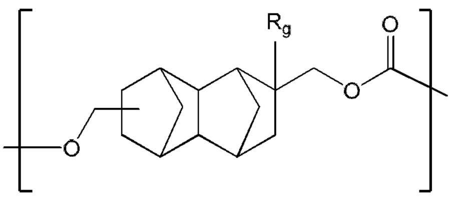

(4)

In the formulae,

$X_a$, $X_b$, $X_c$, $X_d$, $X_e$, and $X_f$ each independently represent an alkylene group having 1 to 4 carbon atoms,

$R_a$, $R_b$, $R_c$, $R_{cc}$, $R_d$, $R_{dd}$, $R_e$, $R_{ee}$, $R_f$, and $R_{ff}$ are each independently selected from a halogen atom, an alkyl group having 1 to 20 carbon atoms, an alkoxy group having 1 to 20 carbon atoms, a cycloalkyl group having 5 to 20 carbon atoms, a cycloalkoxy group having 5 to 20 carbon atoms, an aryl group having 6 to 20 carbon atoms, a heteroaryl group having 3 to 20 carbon atoms and containing one or more heterocyclic atoms selected from O, N, and S, an aryloxy group having 6 to 20 carbon atoms, and $-C{\equiv}C-R_i$,

$R_i$ represents an aryl group having 6 to 20 carbon atoms or a heteroaryl group having 3 to 20 carbon atoms and containing one or more heterocyclic atoms selected from O, N and S,

a, b, c, d, e, and f each independently represent an integer from 0 to 10,

h, i, j, j', k, k', m, m', n, and n' each independently represent an integer from 0 to 4, and

each $R_g$ independently represents a hydrogen atom or an alkyl group having 1 to 3 carbon atoms.

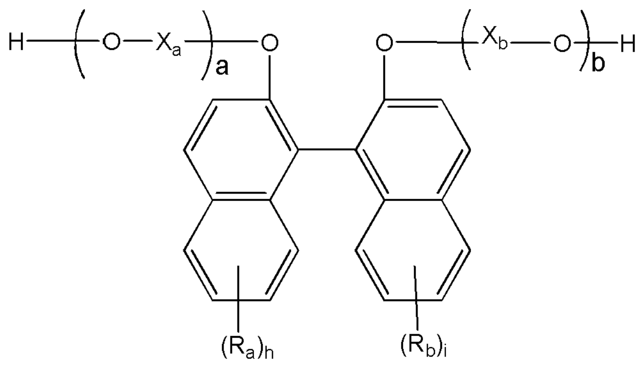

(1')

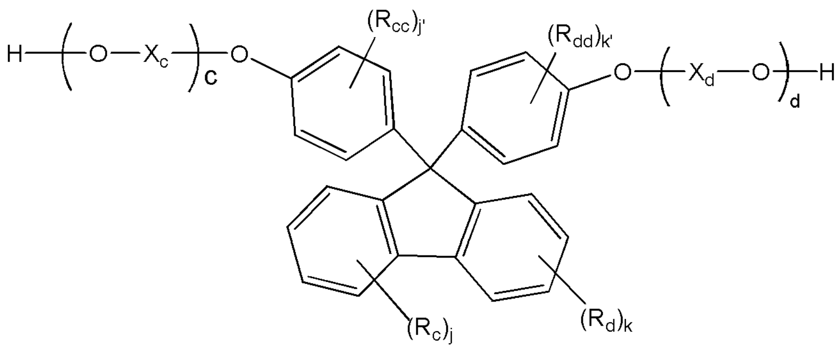

(2')

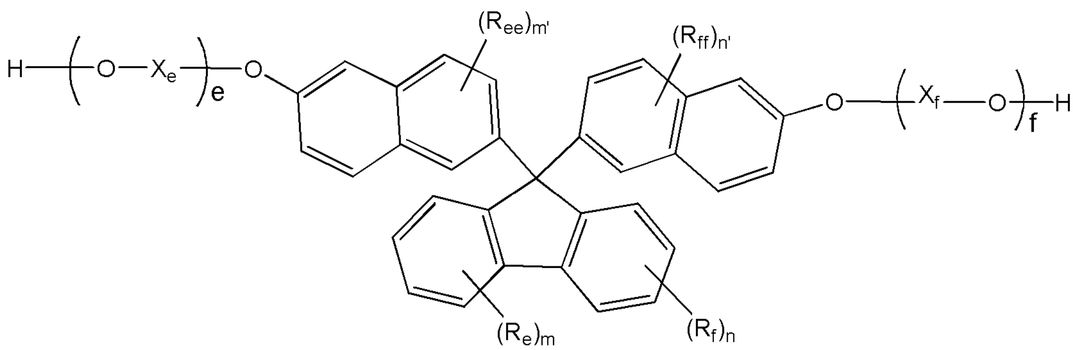

(3')

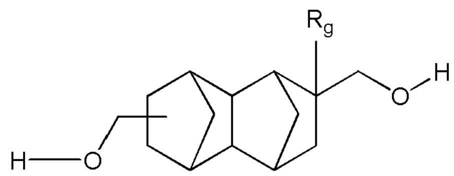

(4')

In the formulae, each symbol represents the same as in the general formulae (1) to (4) set forth above.

According to the manufacturing method described above, a dihydroxy compound derived from a desired synthetic resin can be manufactured from a waste resin composition containing organic impurities and a desired synthetic resin, specifically, a resin having at least two constitutional units selected from the group consisting of the general formulae (1) to (4).

Specifically, first, organic impurities can be suitably removed by the manufacturing method described above. In the step (a1), at least a part of a desired synthetic resin is depolymerized under alkaline conditions. At this time, the organic impurities can be removed from the waste resin composition because the organic impurities contained in the waste resin composition have different chemical properties (ease of depolymerization in an alkaline aqueous solution) and/or physical properties after depolymerization.

When the desired synthetic resin contains two or more types of constitutional units, a mixture of these two or more types of dihydroxy compounds may be obtained after depolymerization. On this occasion, the two or more types of

dihydroxy compounds may be difficult to be separated from each other due to the reason that the two or more types of dihydroxy compounds have similar structures and thus have similar physical properties. However, according to the manufacturing method described above, these dihydroxy compounds can be separated and refined, so that a dihydroxy compound having high purity can be manufactured.

In other words, according to the manufacturing method described above, a high purity dihydroxy compound can be manufactured from a waste resin composition. As a result of this, a high quality resin (recycled resin) can be manufactured when using an obtained dihydroxy compound to manufacture a resin (recycled resin). This means that a waste resin composition can be suitably recycled.

Note that, in one embodiment, the manufacturing method described above may further include a step of preparing a waste resin composition. In one embodiment, the manufacturing method described above includes a step of preparing a waste resin composition, a step (a1), and a step (b1) in this order. Hereinafter, each step will be described in detail.

[Step of preparing waste resin composition]

The first embodiment may include a step of preparing a waste resin composition. On this occasion, the step of preparing a waste resin composition is typically performed before the step (a1).

In one embodiment, the step of preparing a waste resin composition includes comminuting a waste resin composition feedstock to prepare a waste resin composition. In one embodiment, the step of preparing a waste resin composition includes removing a metal from a waste resin composition feedstock. In the step of preparing a waste resin composition, both the comminution and the metal removal may be performed. The order of the comminution and the metal removal in this case is not particularly limited, however, it is preferable that the metal removal is carried out after the comminution, this is because the metal removal can be efficiently performed.

(Waste resin composition feedstock)

The waste resin composition feedstock is not particularly limited, and examples thereof include molded articles collected after having been used as parts of products in the market, defective products generated in molding processes, molded products (sprues, runners, gates, etc.) concomitantly generated in molding processes, defective products generated in productization processes, and those derived from unused molded articles that are no longer needed. Among them, defective products generated in molding processes, molded products (sprues, runners, gates, etc.) concomitantly generated in molding processes, defective products generated in productization processes, and those derived from unused molded articles that are no longer needed are preferable in terms of less deterioration of the desired synthetic resin; and defective products generated in molding processes, molded products (sprues, runners, gates, etc.) concomitantly generated in molding processes are more preferable in terms efficiency in obtaining them. Note that the above-mentioned molded products and the like may be selected, and only those containing organic impurities may be used as the waste resin composition feedstock. For example, sprues may be selected, and those that can be recycled with the status quo may be removed and used for a product, and those that are left containing organic impurities can be used as the waste resin composition feedstock. Moreover, those with different origins may be mixed and used as the waste resin composition feedstock.

The shape of the waste resin composition feedstock is not particularly limited, and examples thereof include: powder, pellets, sheet, film, molded articles and the like, as well as discarded lens, sheet, and film; defective products and burrs generated in manufacturing and/or in molding process; production wastes, solid materials recovered from product wastes using resins, and comminuted products thereof.

The longest diameter of the waste resin composition feedstock is preferably not greater than 100 cm, more preferably not greater than 50 cm, and still more preferably in the range from 0.5 to 3 cm. The waste resin composition feedstock with the longest diameter of not greater than 100 cm is preferable because the energy required for it to be comminuted is reduced. Note that, the term "the longest diameter" as used herein means an average value of the diameters of randomly selected 200 objects, the diameters each having the longest distance whose endpoints lie on the outline of each of the objects.

(Comminution)

The comminution method is not particularly limited, and there may be adopted any method out of compression, impact, shearing, and friction.

Examples of comminutors that can be used include: coarse crushers (jaw crusher, gyratory crusher, impact crusher, uniaxial crusher, biaxial crusher, etc.); secondary crushers (roll crusher, edge runner, disintegrator, semi-autogenous grinding (SAG) mill, crushing roll, hammer mill, roller mill, etc.); fine comminutors (bead mill, ball mill,

vibration ball mill, rod mill, jet mill, planetary mill, etc.) Among them, a coarse crusher is preferably used, and a uniaxial crusher or a biaxial crusher is more preferably used. Specific examples of comminutors include Powerful crushers 35-560, 35-720, 55-770, and 55-1050 (manufactured by TANAKA Co., ltd.), and Low speed granulators KGA-250 and KGA-350 (manufactured by KAWATA MFG. CO., LTD.). Note that, the above-mentioned comminutors may be used singly or in combination of two or more types thereof.

(Metal removal)

Metal removal method is not particularly limited, and examples thereof include method using magnetic force, method using wind force, method using a sieve, method using specific gravity, and method using buoyancy. Among them, a method using magnetic force (method using a magnet, method using a metal detector, etc.), a method using specific gravity, and a method using buoyancy (method using salt water) are preferable. Note that, these methods may be used singly or in combination of two or more types thereof.

Examples of metals to be removed include metals contained in plastic products, metals mingled in molding processes, and metals mingled in comminution processes.

(Waste resin composition)

The waste resin composition feedstock may be used as is for the waste resin composition, but the waste resin composition is preferably those obtained from the waste resin composition feedstock after being subjected to the comminution, metal removal, etc. However, when comminution and metal removal are not required for the waste resin composition feedstock, it is preferable to use the waste resin composition feedstock as is for a waste resin composition. For example, when the waste resin composition feedstock is uniform in size, the longest diameter of the waste resin composition feedstock is small (e.g., the longest diameter is 5 cm or less), or no metal is contained, it is preferable not to perform any step of preparing the waste resin composition in terms of manufacturing cost.

The waste resin composition contains a desired synthetic resin and organic impurities. Note that, the "resin" as used herein means those with a weight average molecular weight of 1000 or more. Moreover, the "weight average molecular weight (Mw)" as used herein means a weight average molecular weight in terms of polystyrene by gel permeation chromatography (GPC).

Desired synthetic resin

The desired synthetic resin is a resin including at least two constitutional units selected from the group consisting of general formulae (1) to (4) set forth below.

Note that, the resin including the constitutional units is typically a polycarbonate (PC) resin or a polyester carbonate resin, preferably a polycarbonate resin.

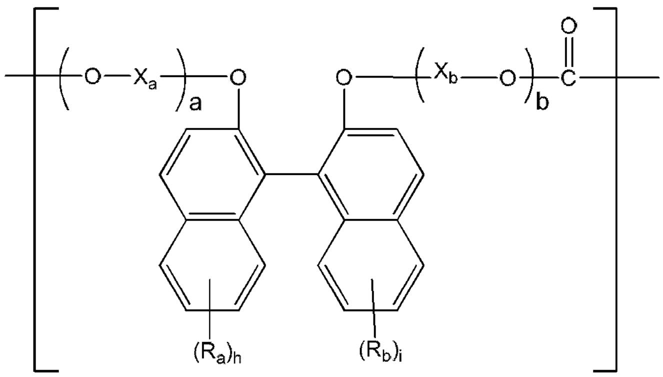

(1)

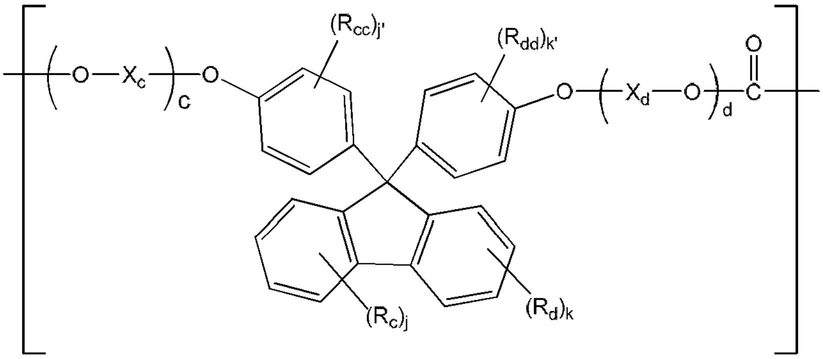

(2)

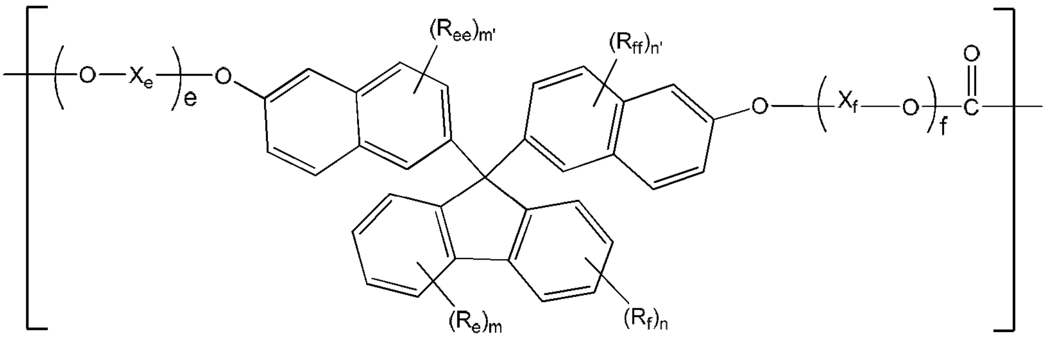

(3)

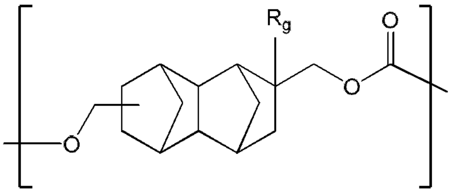

(4)

In the above formulae, $X_a$, $X_b$, $X_c$, $X_d$, $X_e$, and $X_f$ each independently represent an alkylene group having 1 to 4 carbon atoms. Examples of the alkylene group having 1 to 4 carbon atoms include methylene, ethylene, propylene, isopropylene, butylene, isobutylene, sec-butylene, and tert-butylene. Among them, methylene and ethylene are preferable, and ethylene is more preferable.

$R_a$, $R_b$, $R_c$, $R_{cc}$, $R_d$, $R_{dd}$, $R_e$, $R_{ee}$, $R_f$, and $R_{ff}$ are each independently selected from a halogen atom, an alkyl group having 1 to 20 carbon atoms, an alkoxy group having 1 to 20 carbon atoms, a cycloalkyl group having 5 to 20 carbon atoms, a cycloalkoxy group having 5 to 20 carbon atoms, an aryl group having 6 to 20 carbon atoms, a heteroaryl group having 3 to 20 carbon atoms and containing one or more heterocyclic atoms selected from O, N, and S, an aryloxy group having 6 to 20 carbon atoms, and $-C{\equiv}C-R_i$.

Examples of the halogen atom include fluorine atom, chlorine atom, bromine atom, and iodine atom.

Examples of the alkyl group having 1 to 20 carbon atoms include methyl group, ethyl group, propyl group, isopropyl group, butyl group, isobutyl group, sec-butyl group, tert-butyl group, pentyl group, hexyl group, heptyl group, octyl group, nonyl group, decyl group, undecyl group, dodecyl group, and icosyl group.

Examples of the alkoxy group having 1 to 20 carbon atoms include methoxy group, ethoxy group, propyloxy group, isopropyloxy group, butoxy group, isobutoxy group, sec-butoxy group, tert-butoxy group, pentyloxy group, hexyloxy group, heptyloxy group, octyloxy group, nonyloxy group, decyloxy group, undecyloxy group, dodecyloxy group, and icosyloxy group.

Examples of the cycloalkyl group having 5 to 20 carbon atoms include cyclopentyl group, cyclohexyl group, cycloheptyl group, cyclooctyl group, cyclododecyl group, cyclotridecyl group, cyclotetradecyl group, cyclopentadecyl group, cyclooctadecyl group, bicyclo[2.2.1]heptyl group, and bicyclo[2.2.2]octyl group.

Examples of the cycloalkoxy group having 5 to 20 carbon atoms include cyclopentyloxy group, cyclohexyloxy group, cycloheptyloxy group, cyclooctyloxy group, cyclododecyloxy group, cyclotridecyloxy group, cyclotetradecyloxy group, cyclopentadecyloxy group, cyclooctadecyloxy group, bicyclo[2.2.1]heptyloxy group, and bicyclo[2.2.2]octyl group oxy.

Examples of the aryl group having 6 to 20 carbon atoms include phenyl group, tolyl group, xylyl group, trimethylphenyl group, tetramethylphenyl group, ethylphenyl group, ethylmethylphenyl group, diethylphenyl group,

propylphenyl group, isopropylphenyl group, isopropylmethylphenyl group, benzyl group, phenethyl group, phenylpropyl group, naphthyl group, anthracenyl group, phenanthrenyl group, naphthacenyl group, chryserinyl group, pyrenyl group, biphenyl group, terphenyl group, and quaterphenyl group.

Examples of the heteroaryl group having 3 to 20 carbon atoms and containing one or more heterocyclic atoms selected from O, N, and S include: furanyl group, benzofuranyl group, isobenzofuranyl group, pyrrolyl group, imidazolyl group, pyrazolyl group, triazolyl group, pyridyl group, pyrazyl group, pyrimidyl group, pyridazyl group, pyrrolidyl group, indolyl group, isoindolyl group, indazolyl group, quinolyl group, isoquinolyl group, naphthyridyl group, quinoxalyl group, quinazolyl group, pteridyl group, phenanthridyl group, acridinyl group, pyrimidinyl group, phenanthrolinyl group, phenazinyl group, thiophenyl group, thiopyranyl group, benzothiophenyl group, benzothiopyranyl group, oxazolyl group, isoxazolyl group, thiazolyl group, isothiazolyl group, furazanyl group, oxadiazolyl group, dithiazolyl group, benzoxazolyl group, benzisoxazolyl group, benzothiazolyl group, and benzisothiazolyl group.

Examples of the aryloxy group having 6 to 20 carbon atoms include phenyloxy group, tolyloxy group, xylyloxy group, trimethylphenyloxy group, tetramethylphenyloxy group, ethylphenyloxy group, ethylmethylphenyloxy group, diethylphenyloxy group, propylphenyloxy group, isopropylphenyloxy group, isopropylmethylphenyloxy group, naphthyloxy group, anthracenyloxy group, phenanthrenyloxy group, naphthacenyloxy group, chryserinyloxy group, pyrenyloxy group, biphenyloxy group, terphenyloxy group, and quaterphenyloxy group.

Among them, $R_a$, $R_b$, $R_c$, $R_{cc}$, $R_d$, $R_{dd}$, $R_e$, $R_{ee}$, $R_f$, and $R_{ff}$ each are preferably a phenyl group or a naphthyl group, and more preferably a phenyl group, a 1-naphthyl group, or a 2-naphthyl group.

$R_i$ represents an aryl group having 6 to 20 carbon atoms or a heteroaryl group having 3 to 20 carbon atoms and containing one or more heterocyclic atoms selected from O, N, and S. Examples of the aryl group having 6 to 20 carbon atoms and the heteroaryl group having 3 to 20 carbon atoms and containing one or more heterocyclic atoms selected from O, N, and S include those described above. Among them, $R_i$ is preferably a phenyl group or a naphthyl group, and more preferably a phenyl group, a 1-naphthyl group, or a 2-naphthyl group.

The subscripts a, b, c, d, e, and f each independently represent an integer from 0 to 10. In one embodiment, a, b, c, d, e, and f, each independently, are preferably an integer from 0 to 5, more preferably an integer from 0 to 3, and still more preferably 0 or 1. In another embodiment, a, b, c, d, e, and f, each independently, are preferably an integer from 1 to 5, more preferably an integer from 1 to 3, and still more preferably 1 or 2.

The subscripts h, i, j, j', k, k', m, m', n, and n'n each independently represent an integer from 0 to 4, each are preferably an integer from 0 to 3, more preferably an integer from 0 to 2, still more preferably 0 or 1, and particularly preferably 0.

Each $R_g$ independently represents a hydrogen atom or an alkyl group having 1 to 3 carbon atoms. Examples of the alkyl group having 1 to 3 carbon atoms include methyl group, ethyl group, propyl group, and isopropyl group. Among them, it is preferable that each $R_g$ is independently a hydrogen atom.

Specific examples of the constitutional unit represented by the formula (1) include constitutional units derived from 2,2'-bis(1-hydroxymethoxy)-1,1'-binaphthalene, from 2,2'-bis(2-hydroxyethoxy)-1,1'-binaphthalene (also referred to as "BNE"), from 2,2'-bis(2-hydroxyethoxy)-6,6'-diphenyl-1,1'-binaphthalene (also referred to as "DP"), from 2,2'-bis(3-hydroxypropyloxy)-1,1'-binaphthalene, and from 2,2'-bis(4-hydroxybutoxy)-1,1'-binaphthalene. In one embodiment, the constitutional unit represented by the formula (1) contains at least one of constitutional units derived from 2,2'-bis(2-hydroxyethoxy)-1,1'-binaphthalene (BNE) and from 2,2'-bis(2-hydroxyethoxy)-6,6'-diphenyl-1,1'-binaphthalene (DP).

Specific examples of the constitutional units represented by the formula (2) include constitutional units derived from 9,9-bis[4-(2-hydroxyethoxy)phenyl]fluorene (also referred to as "BPEF"), from 9,9-bis[4-(2-hydroxyethoxy)-3-methylphenyl]fluorene, from 9,9-bis[4-(2-hydroxyethoxy)-3-tert-butylphenyl]fluorene, from 9,9-bis[4-(2-hydroxyethoxy)-3-isopropylphenyl]fluorene, from 9,9-bis[4-(2-hydroxyethoxy)-3-cyclohexylphenyl]fluorene, and from 9,9-bis[4-(2-hydroxyethoxy)-3-phenylphenyl]fluorene (also referred to as "BPPEF"). In one embodiment, the constitutional units represented by the formula (2) include at least one of constitutional units derived from a compound selected from 9,9-bis[4-(2-hydroxyethoxy)phenyl]fluorene (BPEF) and 9,9-bis[4-(2-hydroxyethoxy)-3-phenylphenyl]fluorene (BPPEF).

Specific examples of the constitutional units represented by the formula (3) include constitutional units derived from 9,9-bis(hydroxy(poly)alkoxynaphthyl)fluorenes. Examples thereof include constitutional units derived from a compound selected from 9,9-bis[6-(1-hydroxymethoxy)naphthalene-2-yl]fluorene (also referred to as "BNEF"), 9,9-bis[6-(2-hydroxyethoxy)naphthalene-2-yl]fluorene, 9,9-bis[6-(3-hydroxypropoxy)naphthalene-2-yl]fluorene, and 9,9-bis[6-(4-hydroxybutoxy)naphthalene-2-yl]fluorene. In one embodiment, the constitutional units represented by the formula (3) include a constitutional unit derived from 9,9-bis[6-(2-hydroxyethoxy)naphthalene-2-yl]fluorene (BNEF).

Specific examples of the constitutional units represented by the formula (4) include constitutional units derived from decahydro-1,4:5,8-dimethanonaphthalenediols (also referred to as "D-NDM"). Examples thereof include constitutional units derived from a compound selected from (decahydro-1,4:5,8-dimethanonaphthalene-2,6-diyl)dimethanol, (decahydro-1,4:5,8-dimethanonaphthalene-2,7-diyl)dimethanol, (2-methyldecahydro-1,4:5,8-dimethanonaphthalene-2,6-diyl)dimethanol, (2-methyldecahydro-1,4:5,8-dimethanonaphthalene-2,7-diyl)dimethanol, (2-ethyldecahydro-1,4:5,8-dimethanonaphthalene-2,6-diyl)dimethanol, and (2-ethyldecahydro-1,4:5,8-dimethanonaphthalene-2,7-diyl)dimethanol.

At least two of the above-mentioned constitutional units are included in the desired synthetic resin. On this occasion, the constitutional unit may contain two or more types of constitutional units represented by the same general formula (e.g., two types of constitutional units represented by the general formula (1)), or may contain two or more types of constitutional units represented by different general formulae (e.g., one or more types of the constitutional unit represented by the general formula (1) and one or more types of the constitutional unit represented by the general formula (2)). In addition, the above-mentioned constitutional unit may be combined with a constitutional unit of another polycarbonate resin, or may be combined with a constitutional unit of another resin (polyolefin resin, polyester resin) or the like.

The weight average molecular weight (Mw) of the desired synthetic resin is not particularly limited, but is preferably in the range from 10000 to 70000, and more preferably from 15000 to 50000. A desired synthetic resin having a weight average molecular weight (Mw) of 10,000 or more is preferable because, for example, it can retain an appropriate strength when formed into a molded body, such as a resin for optical lenses. On the other hand, a desired synthetic resin having a weight average molecular weight (Mw) of 70,000 or less is preferable because it can retain an appropriate fluidity during resin molding and its moldability can be improved.

The content of the desired synthetic resin in the waste resin composition is preferably 80% by mass or more, and more preferably in the range from 80 to 99% by mass with respect to the mass of the waste resin composition. The content of the desired synthetic resin of 80% by mass or more is preferable because the recycling efficiency is increased.

Organic impurities

Examples of the organic impurities include impurity resins and impurity compounds.

Examples of the impurity resin include thermoplastic resins other than a desired synthetic resin.

The thermoplastic resin is not particularly limited, and examples thereof include polyolefin resins (polyethylene (PE), polypropylene (PP), polyvinyl chloride (PVC), polystyrene (PS), polyvinyl acetate (PVAc), polytetrafluoroethylene (PTFE), etc.), polyurethane (PU) resins, acrylic resins, polyester resins (polyethylene terephthalate (PET), polybutylene terephthalate (PBT)), polyamide (PA) resins, polyacetal resins, cyclic polyolefins (cycloolefin polymers), polyphenylene sulfide (PPS) resins, polysulfone resins, polyether sulfone resins, liquid crystal polymers (LCP), polyetheretherketone (PEEK) resins, polyamideimide (PAI) resins, and copolymers of constitutional units of these resins (acrylonitrile-styrene copolymer resin (AS resin), acrylonitrile-butylene-styrene copolymer resin (ABS resin), etc.)

Among them, the waste resin composition preferably contains an impurity resin having at least one constitutional unit selected from the group consisting of general formulae (6) to (8) set forth below. Note that the impurity resin having the constitutional unit is typically a cyclic polyolefin.

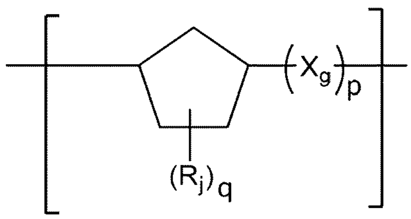

(6)

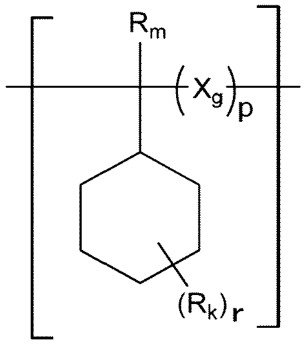

(7)

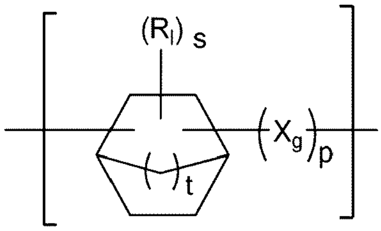

(8)

In the formulae set forth above, each $X_g$ independently represents an alkylene group having 1 to 10 carbon atoms. Examples of the alkylene group having 1 to 10 carbon atoms include methylene, ethylene, propylene, isopropylene, butylene, isobutylene, sec-butylene, tert-butylene, and pentylene. Among them, methylene, ethylene, propylene, butylene, isobutylene, and sec-butylene are preferable, and methylene, ethylene, and propylene are more preferable.

Each of $R_j$, $R_k$, and $R_l$ is independently selected from a halogen atom, a substituted or unsubstituted alkyl group having 1 to 20 carbon atoms, a substituted or unsubstituted alkoxy group having 1 to 20 carbon atoms, a substituted or unsubstituted cycloalkyl group having 5 to 20 carbon atoms, a substituted or unsubstituted cycloalkoxy group having 5 to 20 carbon atoms, a substituted or unsubstituted aryl group having 6 to 20 carbon atoms, a substituted or unsubstituted heteroaryl group having 3 to 20 carbon atoms and containing one or more heterocyclic atoms selected from O, N, and S, a substituted or unsubstituted aryloxy group having 6 to 20 carbon atoms, and $-C\equiv C-R_i$. Examples of $R_j$, $R_k$, and $R_l$ include those similar to $X_a$, $X_b$, $X_c$, $X_d$, $X_e$, and $X_f$ described above.

However, $R_j$, $R_k$, and $R_l$ may have a substituent. The substituent is not particularly limited, and examples thereof include halogen atoms, alkyl groups having 1 to 10 carbon atoms, cycloalkyl groups having 5 to 10 carbon atoms, alkoxy groups having 1 to 10 carbon atoms, cycloalkyloxy groups having 5 to 10 carbon atoms, alkyloxycarbonyl groups having 2 to 10 carbon atoms, cycloalkyloxycarbonyl groups having 5 to 10 carbon atoms, aryloxycarbonyl groups having 7 to 15 carbon atoms, alkylcarbonyloxy groups having 2 to 10 carbon atoms, cycloalkylcarbonyloxy groups having 5 to 10 carbon atoms, arylcarbonyloxy groups having 7 to 15 carbon atoms, hydroxyalkylcarbonyl groups having 2 to 10 carbon atoms, glycidyloxycarbonyl group, hydroxy group, carboxy group, cyano group, and amide groups having 1 to 10 carbon atoms.

Examples of the alkyl group having 1 to 10 carbon atoms include methyl group, ethyl group, propyl group, isopropyl group, butyl group, isobutyl group, sec-butyl group, tert-butyl group, and pentyl group.

Examples of the cycloalkyl group having 5 to 10 carbon atoms include cyclopentyl group, cyclohexyl group, cycloheptyl group, bicyclo[2.2.1]heptyl group, and bicyclo[2.2.2]octyl group.

Examples of the alkoxy group having 1 to 10 carbon atoms include methoxy group, ethoxy group, propyloxy group, isopropyloxy group, butoxy group, isobutoxy group, sec-butoxy group, tert-butoxy group, and pentyloxy group.

Examples of the cycloalkyloxy group having 5 to 10 carbon atoms include cyclopentyloxy group, cyclohexyloxy group, bicyclo[2.2.1]heptyloxy group, and bicyclo[2.2.2]octyloxy group.

Examples of the alkyloxycarbonyl group having 2 to 10 carbon atoms include methyloxycarbonyl group, ethyloxycarbonyl group, propyloxycarbonyl group, isopropyloxycarbonyl group, butyloxycarbonyl group, isobutyloxycarbonyl group, sec-butyloxycarbonyl group, and tert-butyloxycarbonyl group.

Examples of the cycloalkyloxycarbonyl group having 5 to 10 carbon atoms include cyclopentyloxycarbonyl group, cyclohexyloxycarbonyl, bicyclo[2.2.1]heptyloxycarbonyl group, and bicyclo[2.2.2]octyloxycarbonyl group.

Examples of the aryloxycarbonyl group having 7 to 15 carbon atoms include phenyloxycarbonyl group, tolyloxycarbonyl group, xylyloxycarbonyl group, trimethylphenyloxycarbonyl group, tetramethylphenyloxycarbonyl group, ethylphenyloxycarbonyl group, ethylmethylphenyloxycarbonyl group, diethylphenyloxycarbonyl group, and naphthyloxycarbonyl group.

Examples of the alkylcarbonyloxy group having 2 to 10 carbon atoms include methylcarbonyloxy group, ethylcarbonyloxy group, propylcarbonyloxy group, isopropylcarbonyloxy group, and butylcarbonyloxy group.

Examples of the cycloalkylcarbonyloxy group having 5 to 10 carbon atoms include cyclopentylcarbonyloxy group, cyclohexylcarbonyloxy group, bicyclo[2.2.1]heptylcarbonyloxy group, and bicyclo[2.2.2]octylcarbonyloxy group.

Examples of the arylcarbonyloxy group having 7 to 15 carbon atoms include phenylcarbonyloxy group, tolylcarbonyloxy group, xylylcarbonyloxy group, trimethylphenylcarbonyloxy group, tetramethylphenylcarbonyloxy group, ethylphenylcarbonyloxy group, ethylmethylphenylcarbonyloxy group, diethylphenylcarbonyloxy group, and naphthylcarbonyloxy group.

Examples of the hydroxyalkylcarbonyl group having 2 to 10 carbon atoms include hydroxymethylcarbonyl group, hydroxyethylcarbonyl group, and hydroxypropylcarbonyl group.

Examples of the amide group having 1 to 10 carbon atoms include methylaminocarbonyl group, ethylaminocarbonyl group, dimethylaminocarbonyl group, and acetylamino group.

The above-mentioned substituents may be included singly or a combination of two or more types thereof may be included.

$R_i$ represents an aryl group having 6 to 20 carbon atoms or a heteroaryl group having 3 to 20 carbon atoms and containing one or more heterocyclic atoms selected from O, N, and S. The above-mentioned $R_i$ is the same as described above.

Each p independently represents an integer of 0 or 1.

Each of q, r, and s independently represents an integer from 0 to 10, preferably from 0 to 5, and more preferably from 0 to 3.

The subscript t represents an integer from 1 to 3 and is preferably 1 or 2.

Here, when q is 2 or more and two $R_j$'s are present at adjacent carbon atoms, the two $R_j$'s may be bound together to form a ring structure. For example, when q is 2 and two $R_j$'s are both substituted or unsubstituted alkyl groups, the general formula (6) may be the formula (6-1) described below, and when q is 2 and two $R_j$'s are a substituted or unsubstituted alkyl and a substituted or unsubstituted cycloalkyl, the general formula (6) may be the formula (6-2), (6-3), or (6-4) described below.

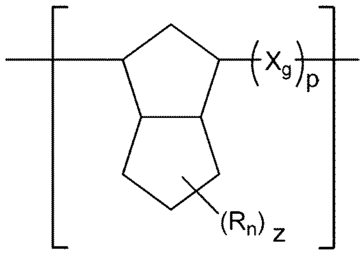

(6-1)

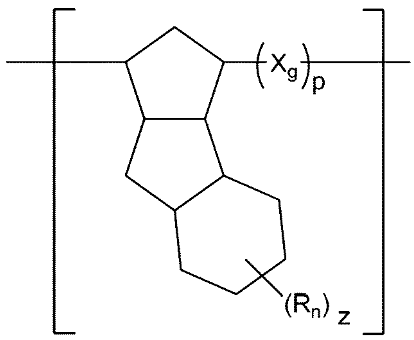

(6-2)

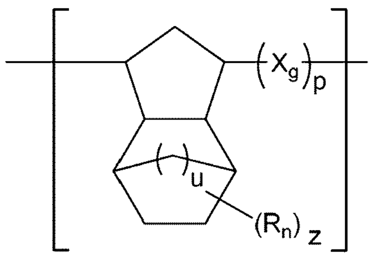

(6-3)

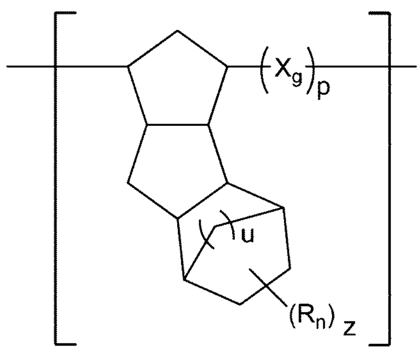

(6-4)

In the formulae set forth above, $X_g$ and p are as mentioned above.

$R_n$ represents the above-mentioned substituent, and specific examples thereof include halogen atoms, alkyl

groups having 1 to 10 carbon atoms, cycloalkyl groups having 5 to 10 carbon atoms, alkoxy groups having 1 to 10 carbon atoms, cycloalkyloxy groups having 5 to 10 carbon atoms, alkyloxycarbonyl groups having 2 to 10 carbon atoms, cycloalkyloxycarbonyl groups having 5 to 10 carbon atoms, aryloxycarbonyl groups having 7 to 15 carbon atoms, alkylcarbonyloxy groups having 2 to 10 carbon atoms, cycloalkylcarbonyloxy groups having 5 to 10 carbon atoms, arylcarbonyloxy groups having 7 to 15 carbon atoms, hydroxyalkylcarbonyl groups having 2 to 10 carbon atoms, glycidyloxycarbonyl group, hydroxy group, carboxy group, cyano group, and amide groups having 1 to 10 carbon atoms.

The subscript z is not particularly limited, but is preferably from 0 to 6, more preferably from 0 to 3, and still more preferably 0 or 1.

The subscript u represents an integer from 1 to 3, and is preferably 1 or 2.

When r is 2 or more and two $R_k$'s are present at adjacent carbon atoms, the two $R_k$'s may be bound together to form a ring structure. For example, when r is 2 and two $R_k$'s are both substituted or unsubstituted alkyl groups, the general formula (7) may be the formula (7-1) or (7-2) set forth below, and when r is 2 and two $R_k$'s are a substituted or unsubstituted alkyl and a substituted or unsubstituted cycloalkyl, the general formula (7) may be the formula (7-3) set forth below.

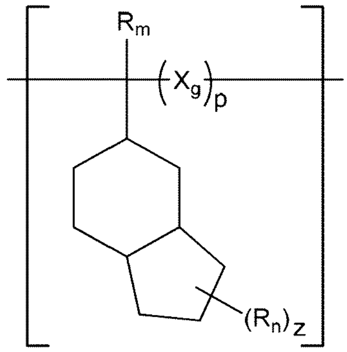

(7-1)

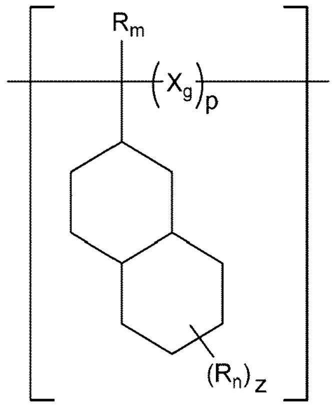

(7-2)

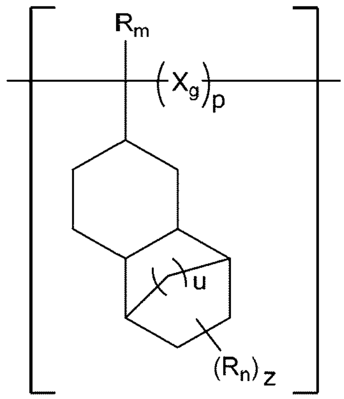

(7-3)

In the formulae set forth above, $X_g$, p, $R_n$, z, and u are as mentioned above.

Furthermore, when s is 2 or more and two Ri's are present at adjacent carbon atoms, the two $R_l$'s may be bound together to form a ring structure. For example, when s is 2 and two $R_l$'s are both substituted or unsubstituted alkyl groups,

the general formula (8) may be the formula (8-1) or (8-2) set forth below, and when s is 2 and two Ri's are a substituted or unsubstituted alkyl and a substituted or unsubstituted cycloalkyl, the general formula (8) may be the formula (8-3) or (8-4) set forth below.

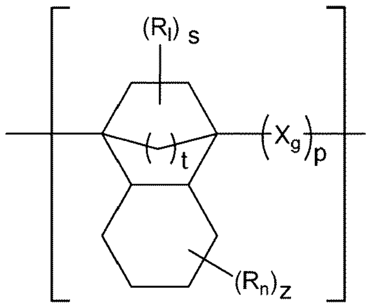

(8-1)

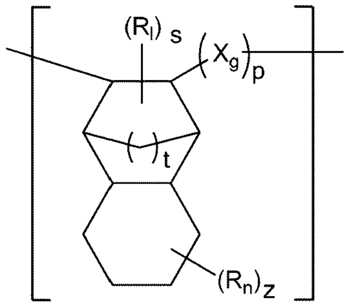

(8-2)

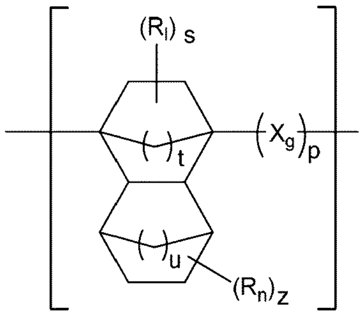

(8-3)

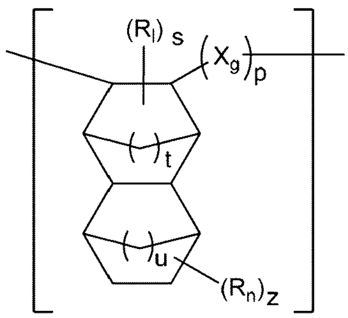

(8-4)

In the formulae set forth above, $X_g$, p, $R_n$, z, and u are as mentioned above.

$R_m$ represents a hydrogen atom or an alkyl group having 1 to 3 carbon atoms. The alkyl group having 1 to 3 carbon atoms is not particularly limited, and examples thereof include methyl group, ethyl group, propyl group, and isopropyl group.

Specific examples of the impurity resin include those containing at least one selected from the group consisting of constitutional units represented by formulae 1 to 8 set forth below.

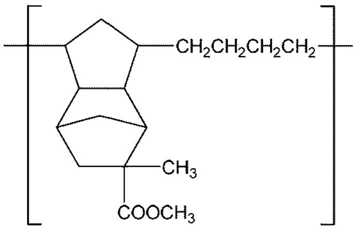

1

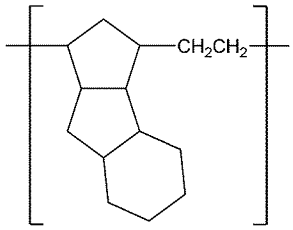

2

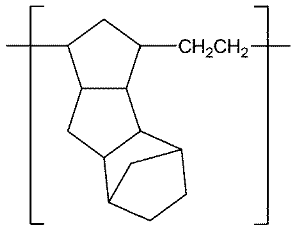

3

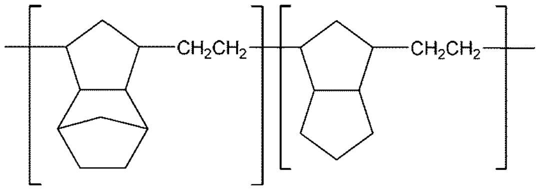

4

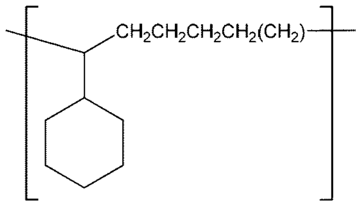

5

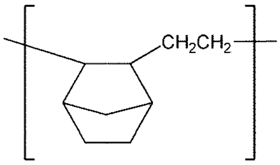

6

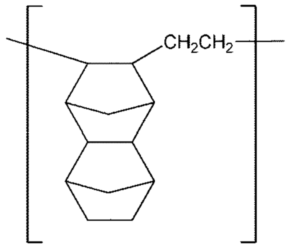

7

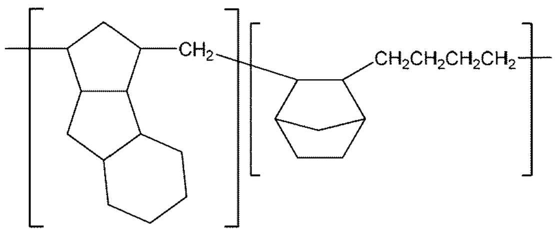

8

The above-described constitutional unit may be contained singly or a combination of two or more types thereof may be contained in the impurity resin. In addition, the above-mentioned constitutional unit may be combined with a constitutional unit of another cyclic polyolefin, or may be combined with a constitutional unit of another resin (polyolefin resin, polyester resin) or the like.

The weight average molecular weight (Mw) of the impurity resin is not particularly limited, but is preferably in the range from 1,000 to three million, more preferably from 10,000 to three million, still more preferably from 20,000 to one million, and particularly preferably from 30,000 to 500,000. An impurity resin having a weight average molecular weight (Mw) of 1,000 or more is preferable because its separation is facilitated. On the other hand, an impurity resin having a weight average molecular weight (Mw) of three million or less is preferable because it is less likely to be a generation source of gel impurities when a trace amount of the impurity resin is contaminated.

The content of the impurity resin in the waste resin composition is preferably 50% by mass or less, more preferably in the range from 0.001 to 50% by mass, still more preferably from 0.01 to 30% by mass, and particularly preferably from 0.1 to 20% by mass with respect to the total mass of the waste resin composition. The content of the impurity resin of 50% by mass or less is preferable because the efficiency is increased.

The impurity compound is not particularly limited, and examples thereof include: monomers, dimers, copolymers, and oligomers of the above-mentioned impurity resins, aryl alcohols (such as phenols), carbonic acid diesters (such as diphenyl carbonates), desired synthetic resin feedstock monomer-modified products (such as formulae (A-1) and (A-2) set forth below), and desired synthetic resin modified products having a moiety represented by the formula (B-1) or (B-2) set forth below, or the like. Note that, the "impurity compound" as used herein means an impurity of an organic compound having a weight average molecular weight of less than 1000. Therefore, when the weight average molecular weight of the

recycled resin-modified product is 1000 or more, the desired synthetic resin-modified product is categorized into an impurity resin.

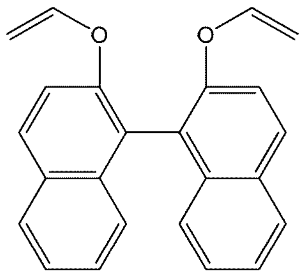

(A-1)

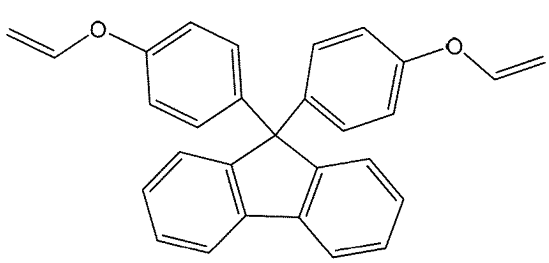

(A-2)

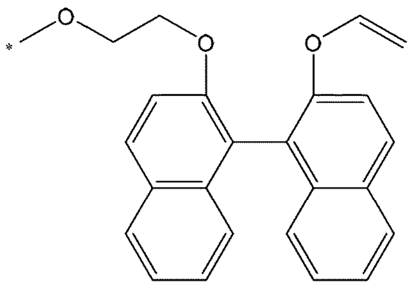

(B-1)

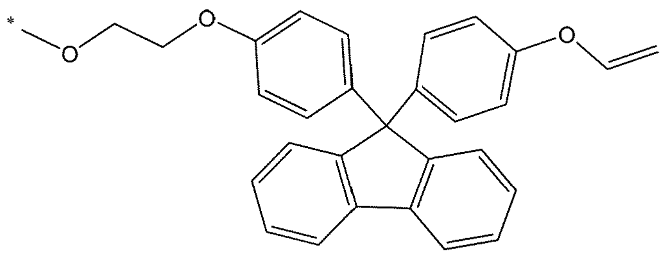

(B-2)

In the formulae (B-1) and (B-2) set forth above, the symbol "*" represents a binding site with a polymer chain.

Shape of waste resin composition

The shape of the waste resin composition is not particularly limited, and examples thereof include powder form, granular form, and rod form.

The longest diameter of the waste resin composition is preferably 5 cm or less, more preferably 3 cm or less, still more preferably in the range from 0.001 to 3 cm, particularly preferably from 0.01 to 2 cm, and very preferably from 0.1 to 1 cm. The waste resin composition the longest diameter of which is 5 cm or less is preferable in terms of easy transportation, easy progression of depolymerization, and the like.

[Step (a1)]

The step (a1) is a step in which an alkaline solution containing a first solvent, water and a waste resin composition containing a resin having at least two constitutional units selected from the group consisting of the general formulae (1) to (4) set forth above is treated to give a reaction solution containing the first solvent and a mixture of dihydroxy compounds containing a first dihydroxy compound selected from the group consisting of the general formulae (1') to (4') set forth below and at least one other dihydroxy compound selected from the group consisting of the general formulae (1') to (4').

(Alkaline solution)

The alkaline solution contains a waste resin composition, a first solvent, and water. The alkaline solution may further contain a metal oxide or the like.

Waste resin composition

The waste resin composition contains a resin having at least two constitutional units selected from the group consisting of the general formulae (1) to (4) set forth above. In addition, the waste resin composition may further contain organic impurities.

Resin

The resin is a resin having at least two constitutional units selected from the group consisting of the general formulae (1) to (4). Because of this, the resin is typically a polycarbonate resin.
Specific examples of the constitutional units represented by the general formulae (1) to (4) are as described above.
The resin may further contain another constitutional unit. This constitutional unit is not particularly limited, and examples thereof include constitutional units derived from olefins and constitutional units derived from esters. The resin may include the other constitutional unit singly or a combination of two or more types of the other constitutional units.
The above-mentioned resin may be contained singly in the waste resin composition, or a mixture of two or more types of the above-mentioned resins may be contained therein.
The weight average molecular weight (Mw) of the resin is not particularly limited, but is preferably in the range from 10000 to 70000, and more preferably from 15000 to 50000. The resin having a weight average molecular weight (Mw) of 10,000 or more is preferable because, for example, it can retain an appropriate strength when formed into a molded body, such as a resin for optical lenses. On the other hand, the resin having a weight average molecular weight (Mw) of 70,000 or less is preferable because it can retain an appropriate fluidity during resin molding and its moldability can be improved.
The content of the resin in the waste resin composition is preferably 80% by mass or more, and more preferably in the range from 80 to 99% by mass with respect to the mass of the waste resin composition. The content of the resin of 80% by mass or more is preferable because the efficiency is increased.

Organic impurities

Examples of the organic impurities include an impurity resin and an impurity compound. The impurity resin and the impurity compound are as mentioned above.
Among them, the impurity resin is preferably a polyolefin resin or a cyclic polyolefin, and more preferably a cyclic polyolefin in terms of that the chemical properties thereof is significantly different (ease of depolymerization in an alkaline aqueous solution thereof is significantly different) from those of the polycarbonate resin.

First solvent

The first solvent has functions to promote depolymerization, to dissolve a dihydroxy compound to be obtained by depolymerization, and the like.
The first solvent is not particularly limited, and examples thereof include aliphatic hydrocarbon-based solvents and aromatic hydrocarbon-based solvents.
The aliphatic hydrocarbon-based solvent is not particularly limited, and examples thereof include pentane, hexane, heptane, octane, nonane, decane, cyclohexane, and cyclodecane.
The aromatic hydrocarbon-based solvent is not particularly limited, and examples thereof include toluene, xylene, and mesitylene.
Among them, the first solvent is preferably an aromatic hydrocarbon-based solvent, and more preferably toluene or xylene. Note that, the above-mentioned reaction solvent may be used singly or a combination of two or more types thereof may be used.
The amount of the first solvent used is not particularly limited, but is preferably in the range from 30 to 2000 parts by mass, more preferably from 40 to 1500 parts by mass, and still more preferably from 100 to 1000 parts by mass with respect to 100 parts by mass of the waste resin composition. The use of the first solvent in an amount of 30 parts by mass or more is preferable because the organic component in the waste resin composition is sufficiently dissolved in the reaction solvent and the reaction efficiency is increased. On the other hand, the use of the first solvent in an amount of 2000 parts by mass or less is preferable because the reaction time is shortened.

Water

Water has a function to promote depolymerization and the like.
The amount of water used is not particularly limited, but is preferably in the range from 10 to 2000 parts by mass, more preferably from 20 to 500 parts by mass, still more preferably from 30 to 3000 parts by mass, and particularly preferably from 40 to 100 parts by mass with respect to 100 parts by mass of the waste resin composition.

Metal oxide

The metal oxide has a function to adjust the reaction solution to alkalinity thereby promoting depolymerization and the like.

The metal oxide is not particularly limited, but examples thereof include: alkali metals, such as sodium hydroxide, potassium hydroxide, and rubidium hydroxide; and alkaline earth metals, such as calcium hydroxide and barium hydroxide. Among them, the metal oxide is preferably an alkali metal, more preferably sodium hydroxide or potassium hydroxide, and still more preferably potassium hydroxide. Note that, single kind of these metal oxides may be used or a combination of two or more types thereof may be used.

The amount of the metal oxide used is not particularly limited, but is preferably in the range from 1.5 to 10 mol, more preferably from 2 to 8 mol, and still more preferably from 2 to 4 mol with respect to 1 mol of the carbonate bond in the polycarbonate resin. The use of the metal oxide in an amount of 1.5 mol or more is preferable because the depolymerization is sufficiently carried out. On the other hand, the use of the metal oxide in an amount of 10 mol or less is preferable because the production cost is reduced.

The concentration of the metal oxide in the alkaline aqueous solution is preferably in the range from 10 to 60% by mass, more preferably from 15 to 55% by mass, and still more preferably from 20 to 50% by mass with respect to the total mass of the aqueous alkaline solution. The concentration of the metal oxide of 10% by mass or more is preferable because the depolymerization reaction rate increases. On the other hand, the concentration of the metal oxide of 60% by mass or less is preferable because the alkaline aqueous solution does not become a slurry and the reaction progresses more easily.

(Treatment)

The treatment of the alkaline solution can afford a reaction solution containing a first solvent and a mixture of dihydroxy compounds containing a first dihydroxy compound selected from the group consisting of the general formulae (1') to (4') and at least one other dihydroxy compound selected from the group consisting of the general formulae (1') to (4').

Note that, organic impurities contained in the waste resin composition are different from the above-mentioned resins in chemical properties in depolymerization. Because of this, the organic impurities either do not react, are depolymerized earlier than the above-mentioned resins, depolymerized later than the above-mentioned resins, or the like. For example, the organic impurities, such as polyolefin resins and cyclic polyolefins, do not usually react under basic conditions, so that only the above-mentioned resins are depolymerized and the organic impurities can be easily removed. Even if the organic impurities are depolymerized, the depolymerized products of the organic impurities can be easily removed because the depolymerized products thereof usually have polarities different from those of the dihydroxy compounds.

As described above, the desired resin includes at least two constitutional units selected from the group consisting of the general formulae (1) to (4). For this reason, the desired resin is depolymerized by the treatment of the alkaline solution, whereby a mixture of at least two types of dihydroxy compounds is obtained. The dihydroxy compounds in the mixture may be similar in structures and hence similar in physical properties and there may be a case where they are difficult to be isolated and purified. As a result, the purity of resultant dihydroxy compounds decreases, and there have been some cases where limitations to recycling have arisen. In contrast, the implementation of the step (b1) described later can manufacture a high purity dihydroxy compound. Note that, the "first dihydroxy compound" is a dihydroxy compound that is intended to be isolated and purified in the step (b1), and the "other dihydroxy compound" is a dihydroxy compound that is not intended to be isolated and purified in the step (b1).

The treatment temperature (depolymerization reaction temperature) is not particularly limited, but is preferably 120°C or lower, more preferably 100°C or lower, and still more preferably in the range from 30 to 90°C. The treatment temperature of 120°C or lower is preferable because side reactions can be prevented.

Incidentally, the solution obtained after the treatment (depolymerization) can be appropriately purified by washing, extraction, or the like, to give a reaction solution. Note that, the solution obtained after the treatment (depolymerization) usually contains an organic phase derived from the first solvent and an aqueous phase derived from water. On this occasion, the mixture of dihydroxy compounds obtained by depolymerization can be contained in the organic phase. For this reason, the aqueous phase derived from water is preferably removed from the solution obtained after the treatment (depolymerization), by liquid-liquid extraction or the like.

In addition, the organic impurities and depolymerized products thereof contained in the obtained reaction solution, when they have physical properties different from those of the dihydroxy compounds, can be appropriately purified and removed at this stage. In this case, the reaction solution and the below-mentioned crystallization solution do not contain, or hardly contain the organic impurities and depolymerized products thereof.

(Reaction solution)

The reaction solution contains a first solvent and a mixture of dihydroxy compounds. The reaction solution may further contain organic impurities, depolymerized products thereof, and the like.

The mixture of dihydroxy compounds contains a first dihydroxy compound selected from the group consisting of the general formulae (1') to (4') set forth above and at least one other dihydroxy compound selected from the group consisting of the general formulae (1') to (4') set forth above.

First dihydroxy compound

The first dihydroxy compound is a dihydroxy compound intended to be isolated and purified in the step (b1).

The first dihydroxy compound is not particularly limited, but is preferably the dihydroxy compound selected from the group consisting of the general formulae (1') to (3'), more preferably the dihydroxy compound of the general formula (1'), and particularly preferably the dihydroxy compound of the general formula (1') (wherein $R_a$ and $R_b$ are phenyl groups, and h and i each are 1).

Note that, the first hydroxy compound preferably has a low polarity, because its solubility in the second solvent at 25°C is smaller than that of the other dihydroxy compounds in the step (b1). For example, the general formula (3') has a skeleton having a fluorene ring with two naphthyl groups, this is preferable because it tends to be difficult to dissolve in the second solvent. A case where in the general formulae (1') to (3'), $R_a$ to $R_f$ include a cycloalkyl group having 5 to 20 carbon atoms, an aryl group having 6 to 20 carbon atoms, or the like is also preferable because it also tends to be difficult to dissolve in the second solvent.

Other dihydroxy compounds

The other dihydroxy compound is a dihydroxy compound that is not intended to be isolated and purified in the step (b1).

The other dihydroxy compound preferably has a high polarity, because its solubility in the second solvent at 25°C is larger than that of the first dihydroxy compound in the step (b1). For example, the general formula (4') is prone to dissolve easily in the second solvent, because it has a smaller number of carbon atoms and hence the polarity thereof is relatively increased as compared with the general formulae (1') to (3').

First solvent

As the first solvent, those described above can be used. Note that, as for the content of the first solvent, the solvent amount of the first solvent can be adjusted by distilling off the solvent or the like in the treatment after depolymerization. By doing this, there can be adjusted the content of the first solvent in the crystallization solution described later.

Organic impurities and depolymerized products thereof

The reaction solution may contain organic impurities and depolymerized products thereof.

The organic impurities are as described above. The organic impurities are greatly different in molecular weight and polarity from the first hydroxy compound, and thus can be easily removed in the step (b1) described later.

The depolymerized products of the organic impurities are not particularly limited, and examples thereof include monomers and oligomers obtained by the depolymerization of the organic impurities. For example, when a polyester resin is contained as an organic impurity, an alcohol and a carboxylic acid can be obtained by depolymerization. These depolymerized products are greatly different in polarity from the dihydroxy compounds, and hence can be easily removed in the step (b1) described later.

[Step (b1)]

The step (b1) is a step of crystallizing the first dihydroxy compound from a crystallization solution obtained by adding a second solvent to the reaction solution obtained in the step (a1).

(Crystallization solution)

The crystallization solution is obtained by adding the second solvent to the reaction solution obtained in the step (a1). Specifically, the crystallization solution contains the first dihydroxy compound, the other dihydroxy compound, and

the first solvent, which are derived from the reaction solution, and a second solvent. The crystallization solution may further contain others, such as a seed crystal, organic impurities, and depolymerized products thereof.

First dihydroxy compound

The first dihydroxy compound is obtained by depolymerization of a desired resin (typically, a polycarbonate resin), and is a dihydroxy compound intended to be isolated and purified. The first dihydroxy compound is the dihydroxy compound selected from the group consisting of the general formulae (1') to (4'), preferably the dihydroxy compound selected from the group consisting of the general formulae (1') to (3'), more preferably the dihydroxy compound of the general formula (1'), and particularly preferably the dihydroxy compound of the general formula (1') (wherein $R_a$ and $R_b$ are phenyl groups, and h and i each are 1). Note that, the first dihydroxy compounds is typically one.

The first dihydroxy compound content by mole (first dihydroxy compound (mol)/mixture of dihydroxy compounds (mol)) in the mixture of dihydroxy compounds (mixture of first dihydroxy compound and other dihydroxy compounds) is preferably 30% by mole or more, preferably in the range from 30 to 95% by mole, more preferably from 40 to 80% by mole, and particularly preferably from 50 to 75% by mole. The first dihydroxy compound content by mole is preferably 30% by mole or more in terms of that a high purity dihydroxy compound can be manufactured, excellent in manufacturing costs, etc.

The content of the first dihydroxy compound in the crystallization solution is preferably in the range from 1 to 35% by mass, more preferably from 5 to 30% by mass, still more preferably from 5 to 25% by mass, and particularly preferably from 10 to 25% by mass with respect to the total mass of the crystallization solution.

Other dihydroxy compounds

The other dihydroxy compound is obtained by the depolymerization of a desired resin (typically, a polycarbonate resin), and is a dihydroxy compound that is not intended to be isolated and purified. Note that, the other dihydroxy compounds may be one type or two or more types.

The other dihydroxy compound content by mole (other dihydroxy compound (mol)/mixture of dihydroxy compounds (mol)) in the mixture of dihydroxy compounds (mixture of first dihydroxy compound and other dihydroxy compound) is preferably 5% by mole or less, preferably in the range from 5 to 70% by mole, more preferably from 20 to 60% by mole, and particularly preferably 25% by mole or more and less than 50% by mole. The first dihydroxy compound content by mole is preferably 5% by mole or less in terms of that a high purity dihydroxy compound can be manufactured, excellent in manufacturing costs, etc. Note that, when two or more types of the other dihydroxy compounds are contained, the total content by mole thereof is preferably in the above-mentioned ranges.

The content of the other dihydroxy compound in the crystallization solution is preferably in the range from 1 to 35% by mass, more preferably from 5 to 30% by mass, still more preferably from 5 to 25% by mass, and particularly preferably from 10 to 25% by mass with respect to the total mass of the crystallization solution. Note that, when two or more types of the other dihydroxy compounds are contained, the total content thereof is preferably in the above-mentioned range.

First solvent

As the first solvent, those described above can be used. The first solvent typically dissolves the first dihydroxy compound and the other dihydroxy compounds.

On this occasion, the solubility of each of the first dihydroxy compound and the other dihydroxy compound in the first solvent at 25°C is preferably greater than or equal to 0.01 g/(10 mL), more preferably is greater than or equal to 0.02 g/(10 mL), and still more preferably is greater than or equal to 0.05 g/(10 mL). Note that, the upper limits of the solubility of the first dihydroxy compound and the other dihydroxy compound in the first solvent at 25°C are not particularly limited, but each are not greater than 3 g/(10 mL), preferably not greater than 2 g/(10 mL), and more preferably not greater than 1 g/(10 mL) in terms of suitably performing crystallization.

The content of the first solvent in the crystallization solution is preferably in the range from 0.1 to 10 g/g, more preferably from 0.5 to 7.5 g/g, and still more preferably from 0.75 to 5 g/g with respect to the total amount of the mixture of the dihydroxy compounds. The content of the first solvent in the above-mentioned range is preferable because a high purity dihydroxy compound (first dihydroxy compound) can be obtained by crystallization. Note that, the content of the first solvent in the crystallization solution can be adjusted by solvent distillation after depolymerization or the like. In addition, the "total amount of the mixture of dihydroxy compounds" means the total amount of the first dihydroxy compound and the other dihydroxy compounds.

Second solvent

In the second solvent, the difference between the solubility of the first dihydroxy compound at 25°C and the solubility of the other dihydroxy compound at 25°C (the difference in solubility at 25°C: the solubility of the other dihydroxy compound (25°C) minus the solubility of the first dihydroxy compound (25°C)) is greater than or equal to 0.1 g/(10 mL), preferably greater than or equal to 0.3 g/(10 mL), and more preferably in the range from 0.4 to 10 g/(10 mL). When a solvent with different solubility at 25°C is added as the second solvent into the crystallization solution, a high purity first dihydroxy compound can be manufactured from a mixture of two or more types of dihydroxy compounds obtained by depolymerization. Note that, when two or more types of the other dihydroxy compounds are contained, the difference in solubility at 25°C is calculated by using the solubility of the type of the other dihydroxy compound less soluble at 25°C among them.

In the second solvent, the difference between the solubility of the first dihydroxy compound at 70°C and the solubility of the other dihydroxy compound at 70°C (the difference in solubility at 70°C: the solubility of the other dihydroxy compound (70°C) minus the solubility of the first dihydroxy compound (70°C)) is preferably greater than or equal to 0.1 g/(10 mL), more preferably greater than or equal to 0.3 g/(10 mL), and particularly preferably in the range from 0.4 to 10 g/(10 mL). The difference in solubility at 70°C of greater than or equal to 0.1 g/(10 mL) is preferable because a high purity dihydroxy compound (first dihydroxy compound) can be efficiently manufactured even on a relatively high temperature side (without cooling).

The second solvent varies depending on the type of the first hydroxy compound and other dihydroxy compounds, but examples thereof include: ketone solvents, such as acetone, methyl ethyl ketone (MEK), methyl isobutyl ketone (MIBK), diisobutyl ketone (DIBK), and cyclohexanone; ether solvents, such as tetrahydrofuran, 1,3-dioxolane, and 1,4-dioxane; and alcohol solvents, such as ethanol, propanol, isopropyl alcohol, butanol, and isobutyl alcohol. Among them, the second solvent is preferably a ketone solvent, and more preferably methyl ethyl ketone (MEK). Note that, single kind of these solvents may be used or a combination of two or more types thereof may be used.

Note that, the first hydroxy compound and the other dihydroxy compound can be determined by selecting the second solvent. For example, when the mixture of dihydroxy compounds includes 2,2'-bis(2-hydroxyethoxy)-6,6'-diphenyl-1,1'-binaphthalene (DP), 2,2'-bis(2-hydroxyethoxy)-1,1'-binaphthalene (BNE), and 9,9-bis[4-(2-hydroxyethoxy)-3-phenylphenyl]fluorene (BPPEF), with reference to the solubility in methyl ethyl ketone (MEK) at 25°C, DP has a solubility of 0.3 g/(10 mL), BNE has a solubility of 0.75 g/(10 mL), and BPPEF has a solubility of 4.5 g/(10 mL). For this reason, when MEK is used as the second solvent, the first hydroxy compound is DP, the other dihydroxy compounds are BNE and BPPEF, and the difference in solubility at 25°C is 0.45 g/(10 mL).

The content of the second solvent in the crystallization solution is preferably in the range from 0.3 to 3 g/g, more preferably from 0.5 to 2.5 g/g, and still more preferably from 0.75 to 2 g/g with respect to the total amount of the mixture of the dihydroxy compounds. The content of the second solvent in the above-mentioned range is preferable because a high purity dihydroxy compound (first dihydroxy compound) can be obtained by crystallization.

The total content of the first solvent and the second solvent in the crystallization solution is preferably in the range from 1 to 10 g/g, more preferably from 1.5 to 7.5 g/g, and still more preferably from 1.5 to 2.5 g/g with respect to the total amount of the mixture of the dihydroxy compounds. The total content of the first solvent and the second solvent of 1 g/g or more is preferable because the purity of the first dihydroxy compound can be increased. On the other hand, the total content of the first solvent and the second solvent of 10 g/g or less is preferable because the yield can be increased.

In one embodiment, the total content of the first solvent and the second solvent in the crystallization solution is preferably 2 g/g or more, and more preferably 4 g/g or more with respect to the total amount of the mixture of the dihydroxy compounds. The upper limit of the total content of the first solvent and the second solvent is preferably 10 g/g or less. The total content of the first solvent and the second solvent of 2 g/g or more is preferable because the purity of the first dihydroxy compound can be increased.

The ratio of the content (g/g) of the first solvent to the content (g/g) of the second solvent in the crystallization solution (first solvent/second solvent) is preferably in the range from 0.8 to 10, more preferably from 0.8 to 7, still more preferably from 0.9 to 4, and particularly preferably from 1 to 3. The ratio (first solvent/second solvent) in the above-mentioned ranges is preferable because the purity of the first dihydroxy compound can be increased.

Seed crystal

The crystallization solution may further contain a seed crystal. The addition of the seed crystal can promote the crystallization of the first dihydroxy compound. Note that, typically, the seed crystal is added to the reaction solution before, in concurrence with, or after the addition of the second solvent.

The seed crystal is not particularly limited, and examples thereof include crystals of the first dihydroxy compound, crystals of the other dihydroxy compounds, and diphenol compounds that can be a feedstock for polycarbonate, such as bisphenol A. Among them, the seed crystal is preferably a crystal of the first dihydroxy compound in view of increasing the purity of the dihydroxy compound (first dihydroxy compound) obtained by crystallization. Note that, single kind of the

above-mentioned seed crystals may be used or a combination of two or more types thereof may be used.

The form of the seed crystal is not particularly limited, but is preferably a crystalline solvate. On this occasion, the compound that forms the crystalline solvate is not particularly limited, but is preferably a dihydroxy compound selected from the group consisting of the general formulae (1') to (3'), and more preferably a dihydroxy compound represented by the general formula (1'). The solvent included in the solvate is not particularly limited, but is preferably the first solvent or the second solvent, and more preferably methanol, toluene, or methyl ethyl ketone. Furthermore, the content of the solvent in the crystalline solvate is preferably in the range from 0.3 to 1.5 mol with respect to 1 mol of the compound that forms the crystalline solvate. By using the seed crystal in the form of a crystalline solvate, a dihydroxy compound having high purity can be manufactured. Note that, the "crystalline solvate" as used herein means a crystal form in which a solvent is contained inside the crystal lattice of the seed crystal.

The ratio of the modal diameter to the median diameter (modal diameter/median diameter) of the seed crystal is preferably 2.0 or less, and more preferably in the range from 1.0 to 1.6. The ratio (modal diameter/median diameter) of 2.0 or less is preferable because the resulting dihydroxy compound can be a high purity and easy to handle massive crystal. Note that, the "modal diameter" as used herein means the most frequently occurring diameter with the value of the maximum frequency, and is determined in particle size measurement by a laser diffraction method. **In** addition, the "median diameter" means the particle diameter corresponding to the cumulative frequency of 50% in a cumulative particle size distribution, and is determined by particle size measurement by a laser diffraction method.

The aspect ratio of the seed crystal is preferably in the range from 1 to 8, and more preferably from 1 to 3. Note that, the "aspect ratio" as used herein means the ratio (L/W) of the maximum crystal length L to the width W in the crystal. **In** this context, the term "maximum crystal length L" means the crystal length that is chosen so the crystal length as to be the longest in a photograph of the crystal taken through an optical microscope. The term "width W" means a length forming an angle of 90 degrees with respect to the maximum crystal length and having the maximum length. Note that, the maximum crystal length L and the width W each are an average value calculated by measuring at least 30 crystals randomly selected from the optical micrograph.

The content of the seed crystal is preferably in the range from 0.001 to 1% by mass, and more preferably from 0.01 to 1% by mass with respect to the total amount of the mixture of the dihydroxy compounds.

Alternatively, the content of the seed crystal is preferably in the range from 0.001 to 1% by mass, more preferably from 0.005 to 0.5% by mass, and still more preferably from 0.01 to 0.1% by mass with respect to the total mass of the crystallization solution.

Organic impurities, depolymerized products thereof

Examples of the organic impurities and depolymerized products thereof include those described above.

The content of the organic impurities in the crystallization solution is preferably in the range from 0.001 to 10% by mass, more preferably from 0.01 to 5% by mass, and still more preferably from 0.1 to 2% by mass with respect to the total mass of the crystallization solution. When two or more types of the organic impurities are contained, the total content thereof is preferably in the above-mentioned ranges.

The content of the depolymerized products of the organic impurities in the crystallization solution is preferably in the range from 0.0001 to 1% by mass, more preferably from 0.001 to 0.5% by mass, and still more preferably from 0.01 to 0.2% by mass with respect to the total mass of the crystallization solution. When two or more types of the depolymerized products of the organic impurities are included, the total content thereof is preferably in the above-mentioned ranges.

(Crystallization)

The first dihydroxy compound can be obtained from the crystallization solution by performing crystallization. On this occasion, the first dihydroxy compound obtained by crystallization is typically in a crystalline form.

In the crystallization, typically, the dihydroxy compounds (the first dihydroxy compound and the other dihydroxy compounds) are preferably heated to be dissolved, and then cooled and crystallized.

The heating temperature before crystallization varies depending on the first solvent and the second solvent to be used, but is preferably in the range from 50 to 90°C, more preferably from 60 to 85°C, and still more preferably from 65 to 80°C.

The crystallization temperature is not particularly limited, but is preferably in the range from -10 to 60°C, more preferably from 0 to 50°C, and still more preferably from 10 to 40°C.

The crystallization time is not particularly limited, but is preferably in the range from 5 minutes to 5 hours, more preferably from 10 minutes to 3 hours, and still more preferably from 30 minutes to 2 hours.

<Second embodiment: Dihydroxy compound manufacturing method>

According to a second embodiment of the present invention, there is provided a method of manufacturing a dihydroxy compound from a waste resin composition. In this context, the method includes: a step (a2) in which an alkaline solution containing a first solvent, water, and a waste resin composition containing a resin having at least two constitutional units selected from the group consisting of general formulae (1) to (4) set forth below is treated to give a reaction solution containing a first solvent and a mixture of dihydroxy compounds containing a first dihydroxy compound selected from the group consisting of general formulae (1') to (4') set forth below and at least one other dihydroxy compound selected from the group consisting of the general formulae (1') to (4'); and a step (b2) in which at least the first dihydroxy compound is crystallized from the reaction solution. Furthermore, the step (b2) includes heating a reaction solution to 80°C or higher.

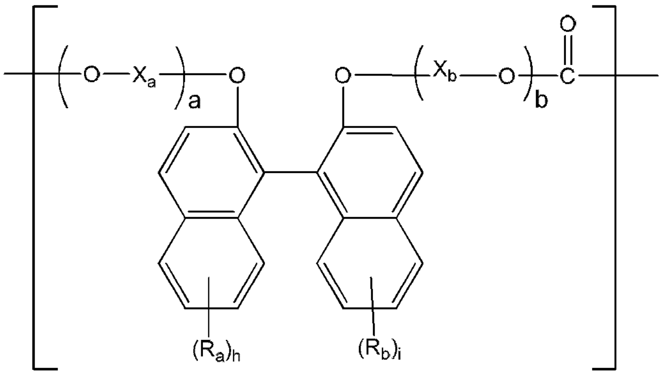

(1)

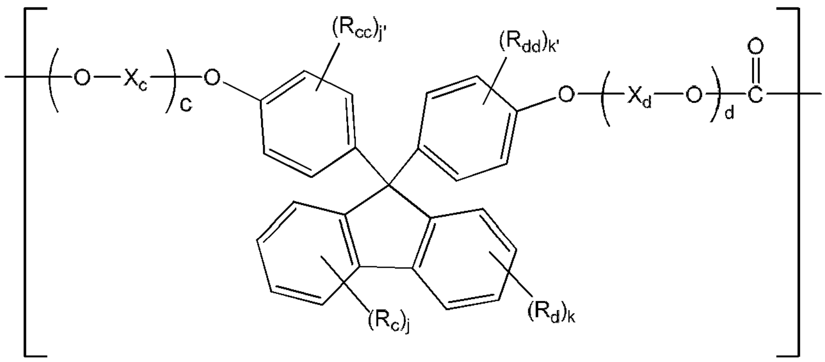

(2)

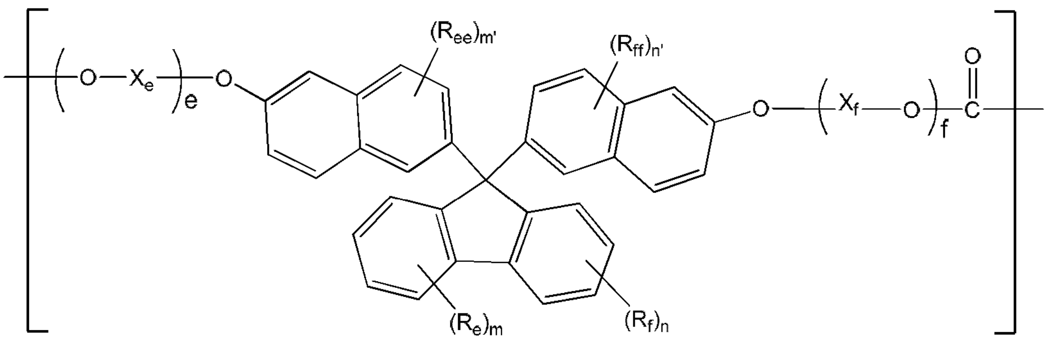

(3)

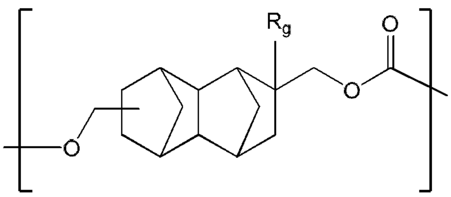

(4)

In the formulae,

$X_a$, $X_b$, $X_c$, $X_d$, $X_e$, and $X_f$ each independently represent an alkylene group having 1 to 4 carbon atoms,
$R_a$, $R_b$, $R_c$, $R_{cc}$, $R_d$, $R_{dd}$, $R_e$, $R_{ee}$, $R_f$, and $R_{ff}$ are each independently selected from a halogen atom, an alkyl group having 1 to 20 carbon atoms, an alkoxy group having 1 to 20 carbon atoms, a cycloalkyl group having 5 to 20 carbon atoms, a cycloalkoxy group having 5 to 20 carbon atoms, an aryl group having 6 to 20 carbon atoms, a heteroaryl group having 3 to 20 carbon atoms and containing one or more heterocyclic atoms selected from O, N, and S, an aryloxy group having 6 to 20 carbon atoms, and $-C{\equiv}C-R_i$,
$R_i$ represents an aryl group having 6 to 20 carbon atoms or a heteroaryl group having 3 to 20 carbon atoms and containing one or more heterocyclic atoms selected from O, N and S,
a, b, c, d, e, and f each independently represent an integer from 0 to 10,
h, i, j, j', k, k', m, m', n, and n' each independently represent an integer from 0 to 4, and
each $R_g$ independently represents a hydrogen atom or an alkyl group having 1 to 3 carbon atoms.

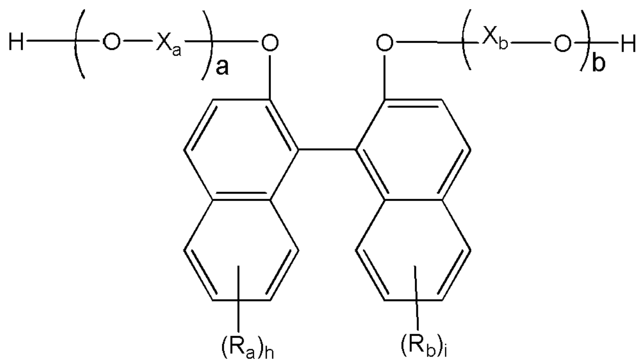

(1')

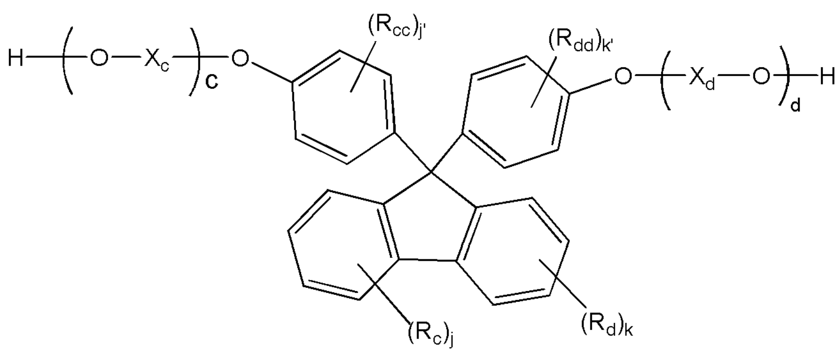

(2')

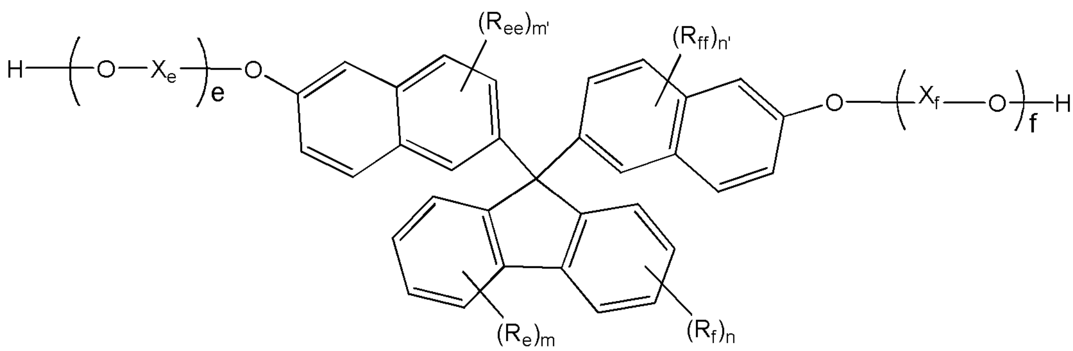

(3')

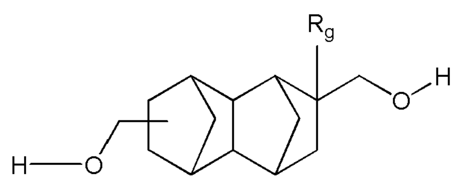

(4')

In the formulae, each symbol represents the same as in the general formulae (1) to (4) set forth above.

According to the manufacturing method described above, a dihydroxy compound derived from a desired

synthetic resin can be manufactured from a waste resin composition containing organic impurities and a desired synthetic resin, specifically, a resin having at least two constitutional units selected from the group consisting of the general formulae (1) to (4).

Specifically, first, as in the first embodiment, organic impurities can be suitably removed. In the step (a2), at least a part of a desired synthetic resin is depolymerized under alkaline conditions. At this time, the organic impurities can be removed from the waste resin composition because the organic impurities contained in the waste resin composition have different chemical properties (ease of depolymerization in an alkaline aqueous solution) and/or physical properties after depolymerization.

When the desired synthetic resin contains two or more types of constitutional units, a mixture of these two or more types of dihydroxy compounds may be obtained after depolymerization. Here, in a case where the dihydroxy compounds are obtained by crystallization, when a solvent (first solvent) suitable for crystallization is selected, there may be a case where at least one of two or more types of dihydroxy compounds does not dissolve sufficiently in the first solvent and the yield may decrease. However, according to the manufacturing method described above, the dihydroxy compounds can be manufactured in high yield.

That is, according to the manufacturing method described above, the dihydroxy compound can be manufactured from the waste resin composition in high yield. This means that a waste resin composition can be suitably recycled.

Note that, in one embodiment, the manufacturing method described above may further include a step of preparing a waste resin composition. In one embodiment, the manufacturing method described above includes a step of preparing a waste resin composition, a step (a2), and a step (b2) in this order. Hereinafter, each step will be described in detail.

[Step of preparing waste resin composition]

The step of preparing a waste resin composition is the same as that described in the first embodiment, and thus the description thereof is omitted here.

[Step (a2)]

The step (a2) is the same as the step (a1) described in the first embodiment, and thus the description thereof is omitted here.

[Step (b2)]

The step (b2) is a step of crystallizing at least the first dihydroxy compound from the reaction solution. At this time, the step (b2) includes heating the reaction solution to 80°C or higher.

(Reaction solution)

The reaction solution contains a first solvent and a mixture of dihydroxy compounds. The reaction solution may further contain organic impurities, depolymerized products thereof, and the like.

The mixture of dihydroxy compounds, the first solvent, the organic impurities, and the depolymerized products thereof are the same as those in the first embodiment, and thus the description thereof is omitted here.

Note that, in the second embodiment, the first hydroxy compound and the other dihydroxy compound can be determined by selecting the first solvent. For example, when the mixture of dihydroxy compounds includes 2,2'-bis(2-hydroxyethoxy)-6,6'-diphenyl-1,1'-binaphthalene (DP), 2,2'-bis(2-hydroxyethoxy)-1,1'-binaphthalene (BNE), and 9,9-bis [4-(2-hydroxyethoxy)-3-phenylphenyl]fluorene (BPPEF), with reference to the solubility in toluene at 25°C, DP has a solubility of 0.3 g/(10 mL), BNE has a solubility of 0.1 g/(10 mL), and BPPEF has a solubility of 0.05 g/(10 mL). Therefore, DP having the highest solubility in toluene is the first dihydroxy compound, and BNE and BPPEF having lower solubility (depending on temperature, there may be a case of not dissolved) in toluene are the other dihydroxy compounds.

In addition, a second solvent and a seed crystal may be further added to the reaction solution. This means that the reaction solution according to the second embodiment may have the same composition as the crystallization solution according to the first embodiment.

The second solvent and the seed crystal are also the same as those of the first embodiment, and thus the description thereof is omitted here.

(Crystallization)

By performing crystallization, at least the first dihydroxy compound can be obtained from the reaction solution. At

this time, the first dihydroxy compound obtained by crystallization may contain another dihydroxy compound. Accordingly, the yield of the resulting dihydroxy compound can be increased.

**In** the crystallization, typically, the dihydroxy compounds (the first dihydroxy compound and the other dihydroxy compounds) are preferably heated to be dissolved, and then cooled and crystallized.

**In** the second embodiment, the heating temperature before crystallization is 80°C or higher, preferably higher than 85°C, and more preferably 90°C or higher. This means that the step (b2) includes heating the reaction solution to 80°C or higher, preferably higher than 85°C, and more preferably 90°C or higher. Note that, the upper limit of the heating temperature before crystallization varies depending on the boiling point of the first solvent, but is preferably 200°C or lower, and more preferably 150°C or lower. Although two or more types of the dihydroxy compounds obtained by depolymerization typically have similar structures and thus have similar physical properties, when a solvent suitable for crystallization (first solvent) is selected, there is a case where the other dihydroxy compound is not dissolved in the first solvent based on the difference in physical properties. Hence, the heating temperature before crystallization is controlled in the step (b2), so that the other dihydroxy compound poorly soluble in the first solvent can be further dissolved. By crystallizing such reaction solution, the yield of the dihydroxy compound can be increased.

The crystallization temperature is not particularly limited, but is preferably in the range from **-10** to 60°C, more preferably from 0 to 50°C, and still more preferably from 10 to 40°C.

The crystallization time is not particularly limited, but is preferably in the range from 5 minutes to 5 hours, more preferably from 10 minutes to 3 hours, and still more preferably from 30 minutes to 2 hours.

<Recycled resin manufacturing method>

According to one embodiment of the present invention, a method of manufacturing a recycled resin is provided. The method of manufacturing a recycled resin includes polymerizing a dihydroxy compound manufactured by the method described above.

In the method for obtaining a recycled resin from a dihydroxy compound, a known polymerization technique is appropriately adopted. In one embodiment, the recycled resin can be manufactured by performing a solution condensation method of a dihydroxy compound and a carbonic acid diester in the presence of a basic compound catalyst and/or a transesterification catalyst or without catalyst. Note that, in terms of adjusting physical properties of the recycled resin and the like, a dihydroxy compound separately prepared can be used as a monomer in combination with a dihydroxy compound manufactured from the waste resin composition.

The carbonic acid diester is not particularly limited, and examples thereof include diphenyl carbonate, ditriel carbonate, bis(chlorophenyl) carbonate, m-cresyl carbonate, dimethyl carbonate, diethyl carbonate, dibutyl carbonate, and dicyclohexyl carbonate. Among them, diphenyl carbonate is preferable.

The amount of the carbonic acid diester compound used is preferably in the range from 0.97 to 1.20 mol, more preferably from 0.98 to 1.10 mol, and still more preferably from 1.00 to 1.10 mol with respect to 1 mol of the dihydroxy compound.

The basic compound catalyst is not particularly limited, and examples thereof include alkali metal compounds, alkaline earth metal compounds, and nitrogen-containing compounds.

The alkali metal compound is not particularly limited, and examples thereof include organic acid salts, inorganic salts, oxides, hydroxides, hydrides, and alkoxides of alkali metals. Specific examples thereof include sodium hydroxide, potassium hydroxide, cesium hydroxide, lithium hydroxide, sodium hydrogen carbonate, sodium carbonate, potassium carbonate, cesium carbonate, lithium carbonate, sodium acetate, potassium acetate, cesium acetate, lithium acetate, sodium stearate, potassium stearate, cesium stearate, lithium stearate, sodium borohydride, sodium phenylborate, sodium benzoate, potassium benzoate, cesium benzoate, lithium benzoate, disodium hydrogen phosphate, dipotassium hydrogen phosphate, dilithium hydrogen phosphate, disodium phenyl phosphate, disodium salts, dipotassium salts, dicesium salts or dilithium salts of bisphenol A, and sodium salts, potassium salts, cesium salts or lithium salts of phenol.

The alkaline earth metal compound is not particularly limited, and examples thereof include organic acid salts, inorganic salts, oxides, hydroxides, hydrides, and alkoxides of alkaline earth metal compounds. Specific examples thereof include magnesium hydroxide, calcium hydroxide, strontium hydroxide, barium hydroxide, magnesium hydrogen carbonate, calcium hydrogen carbonate, strontium hydrogen carbonate, barium hydrogen carbonate, magnesium carbonate, calcium carbonate, strontium carbonate, barium carbonate, magnesium acetate, calcium acetate, strontium acetate, barium acetate, magnesium stearate, calcium stearate, calcium benzoate, and magnesium phenyl phosphate.

The nitrogen-containing compound is not particularly limited, and examples thereof include quaternary ammonium hydroxides, salts thereof, and amines. Specific examples thereof include: quaternary ammonium hydroxides having an alkyl group, an aryl group, or the like, such as tetramethylammonium hydroxide, tetraethylammonium hydroxide, tetrapropylammonium hydroxide, tetrabutylammonium hydroxide, and trimethylbenzylammonium hydroxide; tertiary amines, such as triethylamine, dimethylbenzylamine, and triphenylamine; secondary amines, such as diethylamine and dibutylamine; primary amines, such as propylamine and butylamine; imidazoles, such as 2-methylimidazole, 2-phenylimidazole, and benzimidazole; ammonia, tetramethylammonium borohydride, tetrabutylammonium borohydride, tetrabutylammonium tetraphenylborate, and tetraphenylammonium tetraphenylborate.

Examples of the transesterification catalyst include salts, such as zinc salts, tin salts, zirconium salts, and lead salts. Specific examples thereof include zinc acetate, zinc benzoate, zinc 2-ethylhexanoate, tin(II) chloride, tin(IV) chloride, tin(II) acetate, tin(IV) acetate, dibutyltin dilaurate, dibutyltin oxide, dibutyltin dimethoxide, zirconium acetylacetonate, zirconium oxyacetate, zirconium tetrabutoxide, lead(II) acetate, and lead(IV) acetate.

The basic compound catalyst and the transesterification catalyst described above may be used singly or in combination of two or more types thereof.

The amounts of the basic compound catalyst and the transesterification catalyst used (the total amount when used in combination) are preferably in the range from $1 \times 10^{-9}$ to $1 \times 10^{-3}$ mol, and more preferably from $1 \times 10^{-7}$ to $1 \times 10^{-4}$ mol with respect to 1 mol of the dihydroxy compound.

In melt polycondensation method, a dihydroxy compound and a carbonic acid diester are melted in a reaction vessel, and then the reaction is preferably carried out in a state where a monohydroxy compound produced is retained. In order to retain the monohydroxy compound, the reactor can be closed, depressurized or pressurized to control the pressure.

The recycled resin obtained by the above-described method can be suitably used for applications to plastic products.

## Examples

Hereinafter, the present invention will be described in detail with reference to Examples, but the technical scope of the present invention is not limited thereto. Note that, unless otherwise specified, "parts" and "%" in Examples represent "parts by mass" and "% by mass", respectively.

### [Production example]

As feedstocks, 8.50 kg (16.1 mol) of 2,2'-bis(2-hydroxyethoxy)-6,6'-diphenyl-1,1'-binaphthalene (DP), 1.12 kg (3.0 mol) of 2,2'-bis(2-hydroxyethoxy)-1,1'-binaphthalene (BNE), 6.36 kg (10.8 mol) of 9,9-bis[4-(2-hydroxyethoxy)-3-phenylphenyl]fluorene (BPPEF), 6.62 kg (30.9 mol) of diphenyl carbonate (DPC), and 12 milliliters ($3.0 \times 10^{-4}$ mol, i.e., $10 \times 10^{-6}$ mol with respect to 1 mol of the total of the dihydroxy compounds) of $2.5 \times 10^{-2}$ mol/liter of an aqueous solution of sodium hydrogen carbonate ($NaHCO_3$) were put into a 50 liter reactor equipped with a stirrer and a distillation apparatus, and the reactor was purged with nitrogen, and then heated to 200°C over an hour and stirred under a nitrogen atmosphere of 700 Torr. After 20 minutes from the start of the heating, the complete dissolution of the feedstocks was confirmed, and then the mixture was stirred for 80 minutes under the same conditions. Thereafter, the degree of depressurization was adjusted to 200 Torr over 20 minutes, and the condition of 200 Torr at 200°C was maintained for 10 minutes and a transesterification reaction was carried out. The temperature was raised to 215°C over another 30 minutes, and the pressure was reduced to 180 Torr. Thereafter, the conditions were adjusted to 230°C and 150 Torr over 40 minutes. Thereafter, the pressure was adjusted to 120 Torr over 10 minutes. The conditions were adjusted to 100 Torr and 240°C over another 10 minutes. Thereafter, the pressure was adjusted to 1 Torr over 50 minutes, and polymerization was carried out at 240°C and 1 Torr for 30 minutes. After completion of the reaction, the reactor was pressurized by introducing nitrogen thereinto, and a polycarbonate resin produced was pulled out while being pelletized and the polycarbonate resin was manufactured. Note that, the structural formulae of DP, BNE, BPPEF, and DPC, which are monomer units that make up the polycarbonate resin, are as follows.

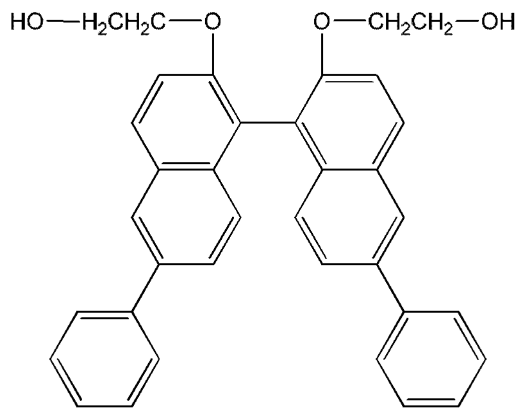

DP

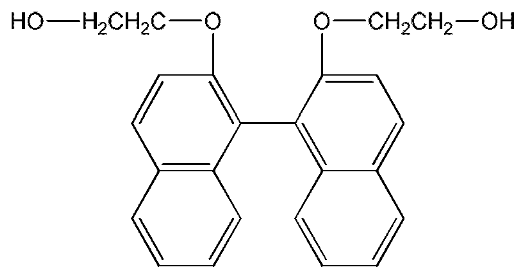

BNE

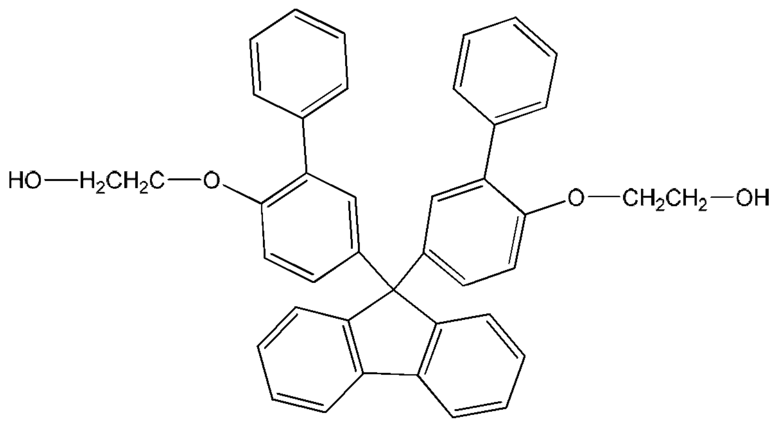

BPPEF

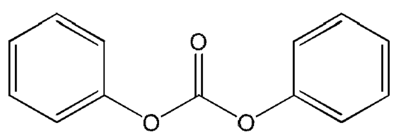

DPC

<First embodiment>

[Example 1-1]

(Step (a1))

Into a reactor equipped with a stirrer and a condenser tube were fed 100 parts by weight of a polycarbonate resin, 88 parts by weight of a 48% sodium hydroxide aqueous solution, and 734 parts by weight of toluene as the first solvent, and

the mixture was allowed to undergo reaction for 3 hours while being heated under reflux. Thereafter, the solution temperature was cooled to 80-85°C, and 178 parts by weight of ion-exchanged water was added thereto. After stirring and standing still, the aqueous phase was separated, and the organic phase was washed with ion-exchanged water. Toluene was partially distilled off from the organic phase to afford a reaction solution.

(Step (b1))

Methyl ethyl ketone (MEK) as the second solvent was added to the reaction solution so as to have the same weight as toluene. Note that MEK-insoluble DP is the first dihydroxy compound, and MEK-soluble BPPEF and BNE are other dihydroxy compounds. After nitrogen purge, the mixed solution was heated and controlled to 70-75°C, and the complete dissolution of the dihydroxy compound was confirmed. Subsequently, the mixed solution was cooled to 40°C, and then DP crystals separately prepared as seed crystals were added to the mixed solution to prepare a crystallization solution.

Crystallization was carried out by cooling the crystallization solution to 30°C and leaving it to stand for at least 1 hour. Filtration was performed, the precipitate was washed with toluene, and the resultant crystals were dried to afford dihydroxy compound crystals. The DP/BPPEF/BNE (mole ratio) in the dihydroxy compound crystals was determined by the method described below.

The purity of the dihydroxy compound crystals was analyzed with high performance liquid chromatography (HPLC). Detailed analysis conditions are as follows.

Apparatus: Agilent Technologies 1260 Infinity
Column: TOSOY TSK-GEL ODS-80-Ts (5 μm, 4.6 mm φ x 250 mm)
Eluents: 0 min acetonitrile/water = 46.3/53.7(% by volume)

: 15 min acetonitrile/water = 46.3/53.7(% by volume)
: 30 min acetonitrile/water = 95/5(% by volume)
: 40 min acetonitrile/water = 95/5(% by volume)

Flow rate: 1 mL/min
Column temperature: 25°C
Measurement wavelength: 254 nm
Injection amount: 5 μm
Sample concentration: 10 mg/(10 mL) (in acetonitrile solvent)

Note that, the crystallization solution (mixture of dihydroxy compounds before crystallization) was measured with HPLC, and the mole ratios of the dihydroxy compounds were calculated from the chromatographic peak areas (%) of the dihydroxy compound crystals based on the obtained chromatographic peak area (%).

DP/BPPEF/BNE (mole ratio) in the dihydroxy compound crystals was found to be 100/0/0. It can be seen from this result that a high purity DP monomer was obtained by using the polycarbonate resin as a feedstock.

[Example 1-2]

In Example 1-1, it was shown that the polycarbonate resin was hydrolyzed into a monomer mixture in the step (a1), and the monomer mixture was able to be isolated in the step (b1).

In Example 1-2 and subsequent examples, in order to examine the conditions of the step (b1), there was used, as the reaction solution to be obtained in the step (a1), a mixed solution imitated this, having the following composition: 5.32 g of DP, 0.7 g of BNE, 3.98 g of BPPEF, and 8.62 g (addition amount per 1 g of the total dihydroxy compound (DP + BNE + BPPEF): 0.86 g) of toluene. On this occasion, DP/BPPEF/BNE (mole ratio) = 54/36/10.

(Step (b1))

The mixed solution was put into a 100 mL recovery flask, and 8.62 g of MEK (addition amount per 1 g of total dihydroxy compound (DP + BNE + BPPEF): 0.86 g) was added thereto. After nitrogen purge, the dihydroxy compounds were completely dissolved while the mixed solution being heated and controlled at 70-75°C. The mixed solution was cooled to 40°C, and then 10 mg of DP separately prepared as seed crystals was added to this mixed solution to prepare a crystallization solution. Note that the dihydroxy compounds each have a solubility in MEK at 25°C set forth below.

$$DP = 0.3\ g/(10\ mL)$$

$$BNE = 0.75\ g/(10\ mL)$$

$$BPPEF = 4.5\ g/(10\ mL)$$

Crystallization was carried out by cooling the crystallization solution to 30°C and leaving it to stand for 1 hour. Filtration was performed, the precipitate was washed with toluene, and the resultant crystals were dried to afford dihydroxy compound crystals. The DP/BPPEF/BNE (mole ratio) in the dihydroxy compound crystals was determined by the same method as in Example 1, and it was found that DP/BPPEF/BNE (mole ratio) = 100/0/0.

The yield was calculated based on the formula set forth below and found to be 79%. Note that the above-mentioned yield is a value calculated by a conversion on the assumption that all the dihydroxy compound crystals are DP.

$$\text{Yield (\%)} = \frac{\text{Weight of dihydroxy compound crystals}}{\text{Weight of DP in crystallization solution}} \times 100$$

[Examples 1-3 to 1-13 and Comparative Example 1-1]

Dihydroxy compound crystals were obtained in the same manner as in Example 1-2 except that the addition amounts of toluene and MEK were modified. The obtained results are shown in Table 1 below.

[Table 1]

| | Toluene equivalent weight (g/g) | MEK equivalent weight (g/g) | Total solvent equivalent weight (g/g) | Toluene/MEK | DP/BPPEF/BNE (mole ratio) | Yield (%) |
|---|---|---|---|---|---|---|
| Example 1-2 | 0.86 | 0.86 | 1.72 | 50/50 | 100/0/0 | 79 |
| Example 1-3 | 1.03 | 1.03 | 2.07 | 50/50 | 100/0/0 | 75 |
| Example 1-4 | 1.38 | 1.38 | 2.76 | 50/50 | 100/0/0 | 68 |
| Example 1-5 | 1.72 | 1.72 | 3.45 | 50/50 | 100/0/0 | 62 |
| Example 1-6 | 1.94 | 1.94 | 3.88 | 50/50 | 100/0/0 | 57 |
| Example 1-7 | 0.69 | 0.69 | 1.38 | 50/50 | 91/7/2 | 65 |
| Example 1-8 | 3.49 | 0.39 | 3.88 | 90/10 | 59/41/0 | 105 |
| Example 1-9 | 3.10 | 0.78 | 3.88 | 80/20 | 73/27/0 | 80 |
| Example 1-10 | 2.72 | 1.16 | 3.88 | 70/30 | 100/0/0 | 58 |
| Example 1-11 | 2.33 | 1.55 | 3.88 | 60/40 | 100/0/0 | 57 |
| Example 1-12 | 4.66 | 0.52 | 5.17 | 90/10 | 87/13/0 | 52 |
| Example 1-13 | 4.14 | 1.03 | 5.17 | 80/20 | 100/0/0 | 46 |
| Comparative Example 1-1 | 5.17 | 0 | 5.17 | 100/0 | 55/44/1 | 135 |

It can be seen from the results in Table 1 that high purity dihydroxy compound crystals with a large amount of DP contained in the dihydroxy compound were obtained in Examples 1-2 to 1-13.

<Second embodiment>

[Example 2-1]

In Example 2-1, in order to examine the conditions of the step (b2), there was used, as the reaction solution to be obtained in the step (a2), a mixed solution imitated this, having the following composition: 5.32 g of DP, 0.70 g of BNE, 3.98

g of BPPEF, and 51.72 g (the addition amount per 1 g of the total dihydroxy compound (DP + BNE + BPPEF): 5.17 g) of toluene. On this occasion, DP/BPPEF/BNE (mole ratio) = 54/36/10. Note that the dihydroxy compounds each have a solubility in toluene at 25°C set forth below.

$$DP = 0.3\ g/(10\ mL)$$

$$BNE = 0.1\ g/(10\ mL)$$

$$BPPEF = 0.05\ g/(10\ mL)$$

(Step (b2))

The mixed solution was put into a 100 mL recovery flask and purged with nitrogen, and then the dihydroxy compounds were completely dissolved while the reaction solution being heated and controlled at 90-95°C. The reaction solution was cooled to 35°C, and then 10 mg of DP separately prepared as seed crystals was added to this reaction solution.

Crystallization was carried out by cooling the reaction solution to 30°C and leaving it to stand for 1 hour. Filtration was performed, the precipitate was washed with toluene, and the resultant crystals were dried to afford dihydroxy compound crystals.

The DP/BPPEF/BNE (mole ratio) in the dihydroxy compound crystals was determined by the same method as in Example 1-1, and it was found that DP/BPPEF/BNE (mole ratio) = 57/42/1. The yield was also calculated by the same method as in Example 1-1 and found to be 137%.

It can be seen from the results of Example 2-1, therefore, that a dihydroxy compound was obtained in high yield.

## Claims

1. A method of manufacturing a dihydroxy compound from a waste resin composition, the method comprising:

   a step (a1) in which an alkaline solution containing a first solvent, water, and a waste resin composition containing a resin having at least two constitutional units selected from the group consisting of general formulae (1) to (4) set forth below:

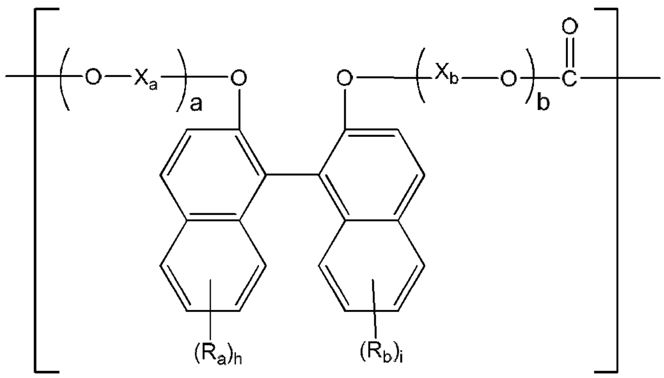

(1)

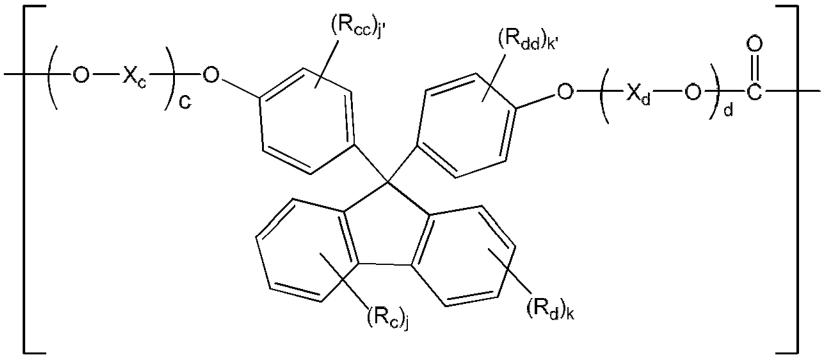

(2)

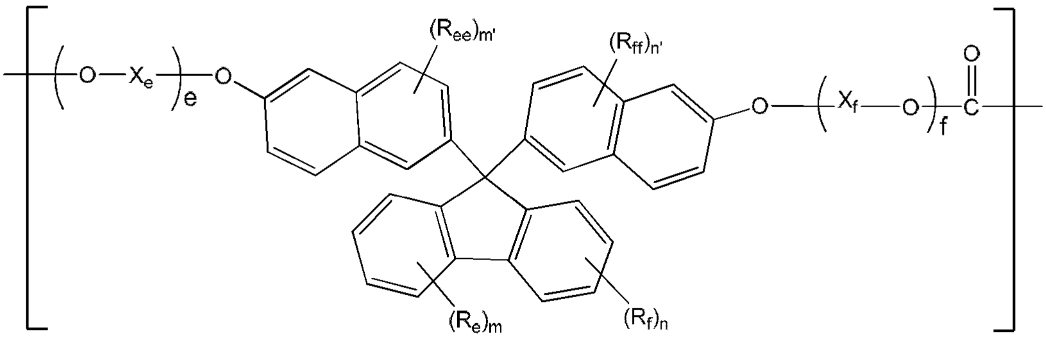

(3)

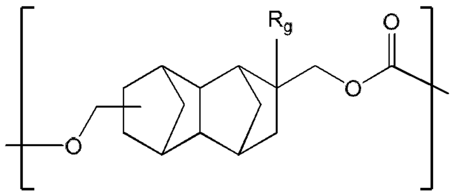

(4)

[wherein,

$X_a$, $X_b$, $X_c$, $X_d$, $X_e$, and $X_f$ each independently represent an alkylene group having 1 to 4 carbon atoms,

$R_a$, $R_b$, $R_c$, $R_{cc}$, $R_d$, $R_{dd}$, $R_e$, $R_{ee}$, $R_f$, and $R_{ff}$ are each independently selected from a halogen atom, an alkyl group having 1 to 20 carbon atoms, an alkoxy group having 1 to 20 carbon atoms, a cycloalkyl group having 5 to 20 carbon atoms, a cycloalkoxy group having 5 to 20 carbon atoms, an aryl group having 6 to 20 carbon atoms, a heteroaryl group having 3 to 20 carbon atoms and containing one or more heterocyclic atoms selected from O, N, and S, an aryloxy group having 6 to 20 carbon atoms, and -C≡C-$R_i$,

$R_i$ represents an aryl group having 6 to 20 carbon atoms or a heteroaryl group having 3 to 20 carbon atoms and containing one or more heterocyclic atoms selected from O, N and S,

a, b, c, d, e, and f each independently represent an integer from 0 to 10, h, i, j, j', k, k', m, m', n, and n' each independently represent an integer from 0 to 4, and

each $R_g$ independently represents a hydrogen atom or an alkyl group having 1 to 3 carbon atoms]

is treated to give a reaction solution containing a first solvent and a mixture of dihydroxy compounds containing a first dihydroxy compound selected from the group consisting of general formulae (1') to (4') set forth below:

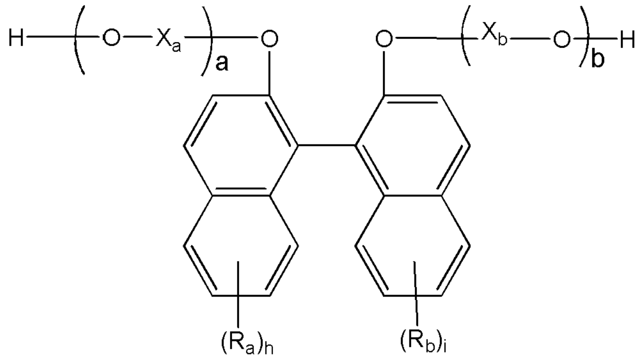

(1')

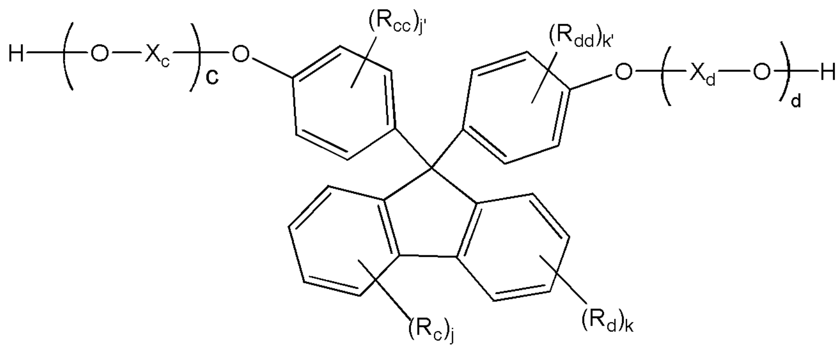

(2')

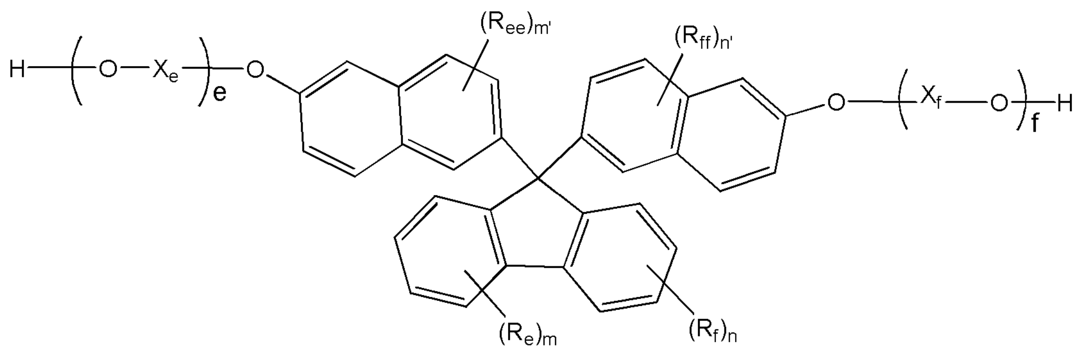

(3')

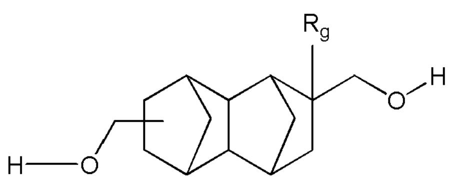

(4')

[wherein, each symbol represents the same as in the general formulae (1) to (4) set forth above]
and at least one other dihydroxy compound selected from the group consisting of the general formulae (1') to (4'); and
a step (b1) in which the first dihydroxy compound is crystallized from a crystallization solution obtained by adding a second solvent to the reaction solution,
wherein a difference between the solubility of the first dihydroxy compound in the second solvent at 25°C and the solubility of the other dihydroxy compound in the second solvent at 25°C (solubility of the other dihydroxy compound minus solubility of the first dihydroxy compound) is greater than or equal to 0.1 g/(10 mL).

2. The method according to claim 1, wherein the crystallization solution further contains a seed crystal.

3. The method according to claim 1 or 2, wherein a content of the first solvent in the crystallization solution is from 0.1 to 10 g/g with respect to a total amount of the mixture of the dihydroxy compounds.

**4.** The method according to any one of claims 1 to 3, wherein a content of the second solvent in the crystallization solution is from 0.3 to 3 g/g with respect to a total amount of the mixture of the dihydroxy compounds.

**5.** The method according to any one of claims 1 to 4, wherein a total content of the first solvent and the second solvent in the crystallization solution is from 1 to 10 g/g with respect to a total amount of the mixture of the dihydroxy compounds.

**6.** The method according to any one of claims 1 to 5, wherein a ratio of a content (g/g) of the first solvent to a content (g/g) of the second solvent in the crystallization solution (first solvent/second solvent) is from 0.8 to 10.

**7.** A method of manufacturing a dihydroxy compound from a waste resin composition, the method comprising:

a step (a2) in which an alkaline solution containing a first solvent, water, and a waste resin composition containing a resin having at least two constitutional units selected from the group consisting of general formulae (1) to (4) set forth below:

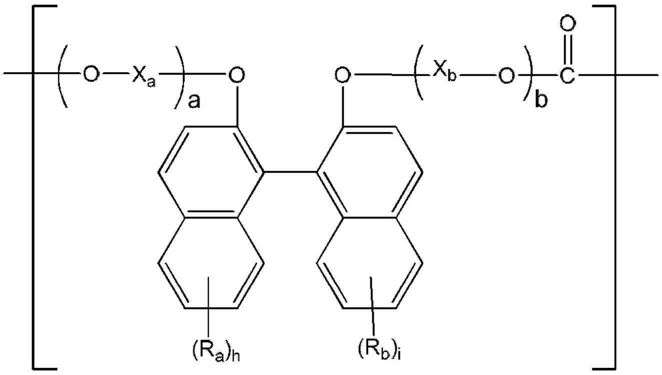

(1)

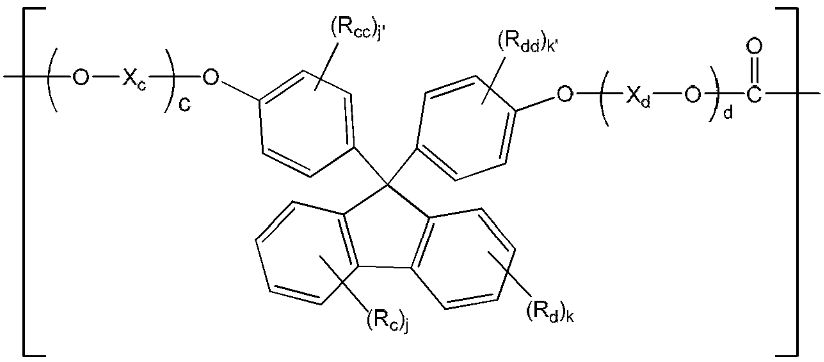

(2)

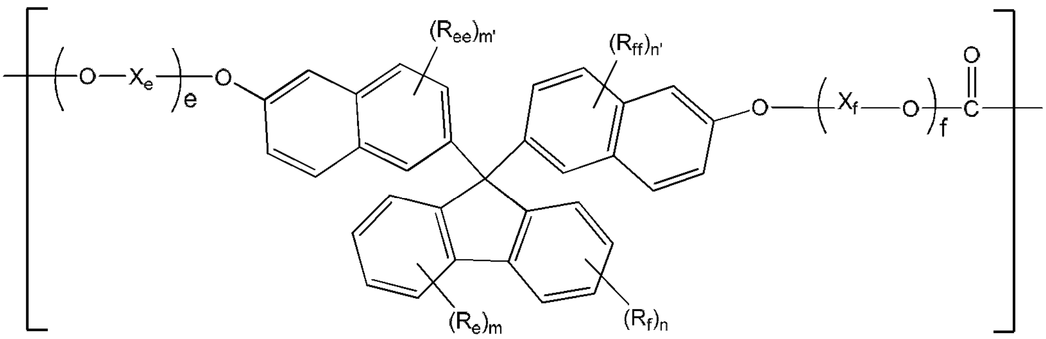

(3)

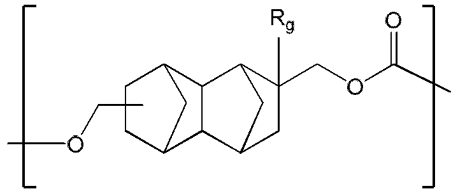

(4)

[wherein,

$X_a$, $X_b$, $X_c$, $X_d$, $X_e$, and $X_f$ each independently represent an alkylene group having 1 to 4 carbon atoms,

$R_a$, $R_b$, $R_c$, $R_{cc}$, $R_d$, $R_{dd}$, $R_e$, $R_{ee}$, $R_f$, and $R_{ff}$ are each independently selected from a halogen atom, an alkyl group having 1 to 20 carbon atoms, an alkoxy group having 1 to 20 carbon atoms, a cycloalkyl group having 5 to 20 carbon atoms, a cycloalkoxy group having 5 to 20 carbon atoms, an aryl group having 6 to 20 carbon atoms, a heteroaryl group having 3 to 20 carbon atoms and containing one or more heterocyclic atoms selected from O, N, and S, an aryloxy group having 6 to 20 carbon atoms, and $-C{\equiv}C-R_i$,

$R_i$ represents an aryl group having 6 to 20 carbon atoms or a heteroaryl group having 3 to 20 carbon atoms and containing one or more heterocyclic atoms selected from O, N and S,

a, b, c, d, e, and f each independently represent an integer from 0 to 10, h, i, j, j', k, k', m, m', n, and n' each independently represent an integer from 0 to 4, and

each $R_g$ independently represents a hydrogen atom or an alkyl group having 1 to 3 carbon atoms]

is treated to give a reaction solution containing a first solvent and a mixture of dihydroxy compounds containing a first dihydroxy compound selected from the group consisting of general formulae (1') to (4') set forth below:

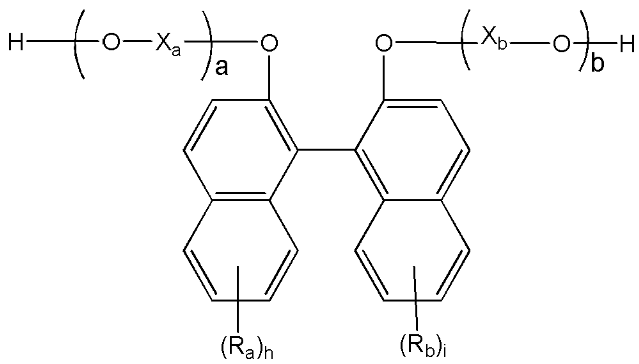

(1')

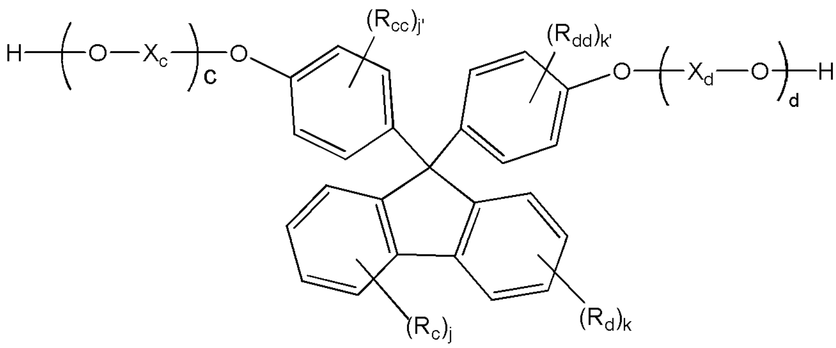

(2')

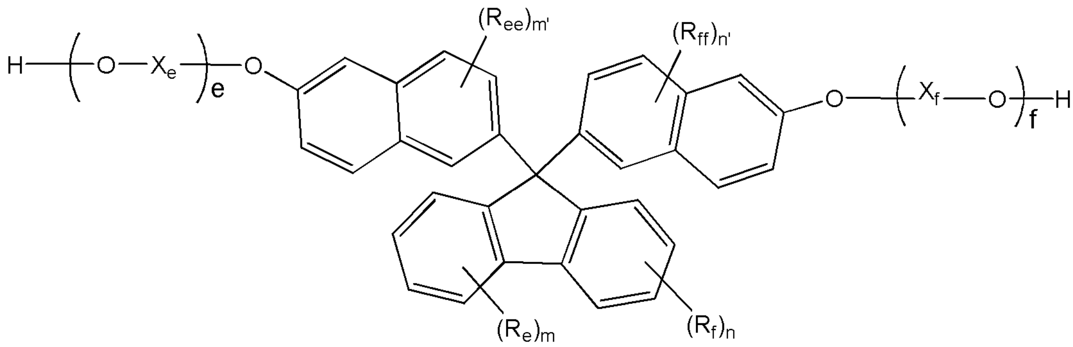

(3')

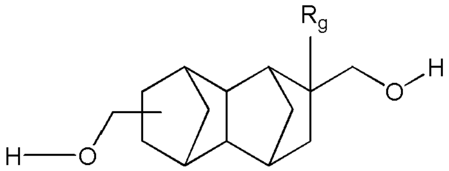

(4')

[wherein, each symbol represents the same as in the general formulae (1) to (4) set forth above]
and at least one other dihydroxy compound selected from the group consisting of the general formulae (1') to (4'); and
a step (b2) in which at least the first dihydroxy compound is crystallized from the reaction solution, wherein the step (b2) comprises heating a reaction solution to 80°C or higher.

**8.** The method according to any one of claims 1 to 7, wherein the solution of the waste resin composition contains an impurity resin having at least one constitutional unit selected from the group consisting of general formulae (6) to (8) set forth below:

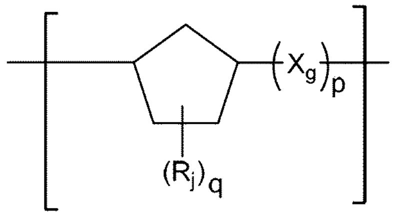

(6)

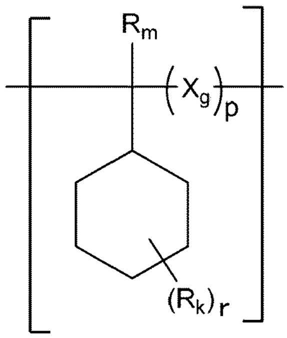

(7)

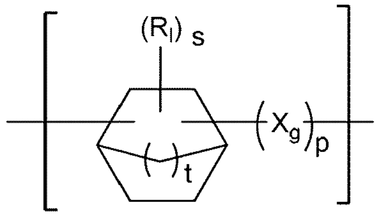

(8)

[wherein,

each $X_g$ independently represents an alkylene group having 1 to 10 carbon atoms;
$R_j$, $R_k$, and $R_l$ are each independently selected from a halogen atom, a substituted or unsubstituted alkyl group having 1 to 20 carbon atoms, a substituted or unsubstituted alkoxy group having 1 to 20 carbon atoms, a substituted or unsubstituted cycloalkyl group having 5 to 20 carbon atoms, a substituted or unsubstituted cycloalkoxy group having 5 to 20 carbon atoms, a substituted or unsubstituted aryl group having 6 to 20 carbon atoms, a substituted or unsubstituted heteroaryl group having 3 to 20 carbon atoms and containing one or more heterocyclic atoms selected from O, N, and S, a substituted or unsubstituted aryloxy group having 6 to 20 carbon atoms, and $-C{\equiv}C-R_i$,
$R_i$ represents a substituted or unsubstituted aryl group having 6 to 20 carbon atoms or heteroaryl group having 3 to 20 carbon atoms and containing one or more heterocyclic atoms selected from O, N, and S,
each p independently represents an integer of 0 or 1,
q, r, and s each independently represent an integer from 0 to 10,
t represents an integer from 1 to 3,
wherein when q is 2 or more and two $R_j$'s are present at adjacent carbon atoms, the two $R_j$'s may be bound together to form a ring structure,
when r is 2 or more and two $R_k$'s are present at adjacent carbon atoms, the two $R_k$'s may be bound together to form a ring structure,
when s is 2 or more and two Ri's are present at adjacent carbon atoms, the two $R_l$'s may be bound together to form a ring structure, and
$R_m$ represents a hydrogen atom or an alkyl group having 1 to 3 carbon atoms].

9. A method of manufacturing a recycled resin, comprising polymerizing a dihydroxy compound manufactured by the method according to any one of claims 1 to 8.

**Patentansprüche**

1. Verfahren zur Herstellung einer Dihydroxyverbindung aus einer Abfallharzzusammensetzung, wobei das Verfahren umfasst:

einen Schritt (a1), in dem eine alkalische Lösung, die ein erstes Lösungsmittel, Wasser und eine Abfallharzzusammensetzung enthält, die ein Harz mit mindestens zwei Struktureinheiten enthält, die ausgewählt sind aus der Gruppe bestehend aus den unten angegebenen allgemeinen Formeln (1) bis (4):

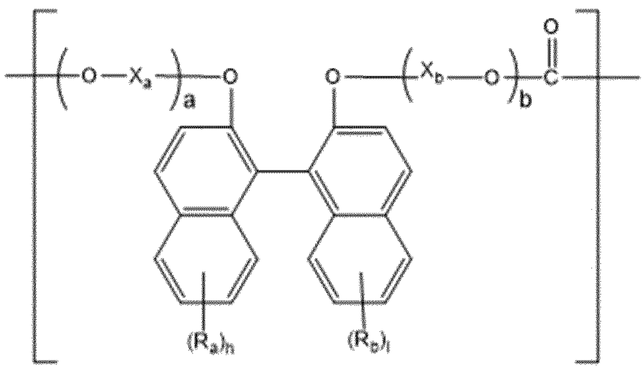

(1)

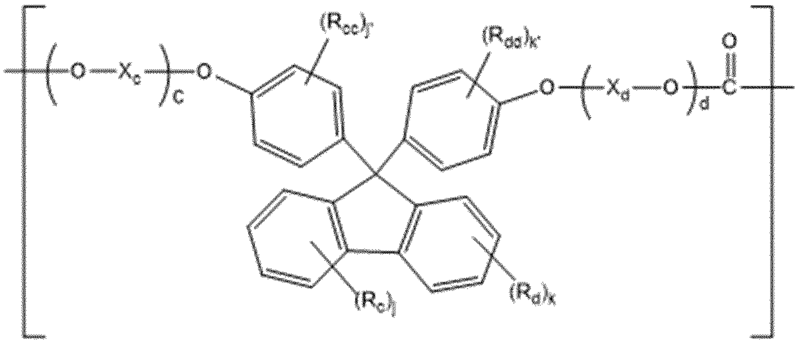

(2)

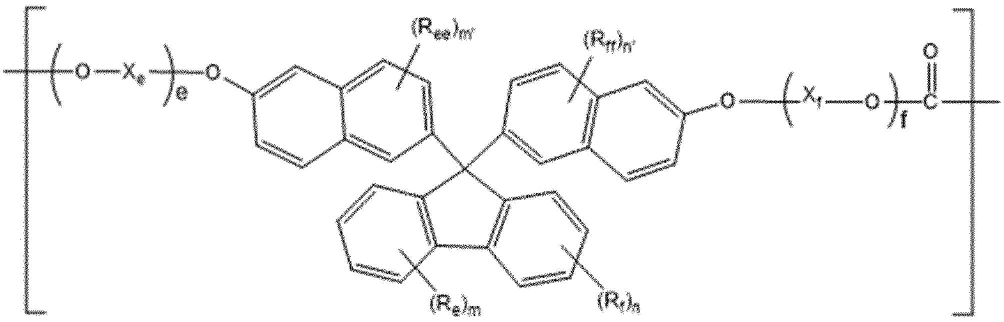

(3)

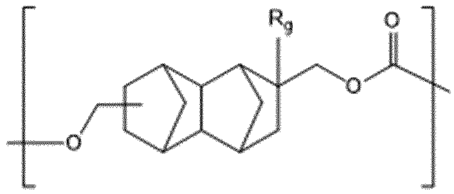

(4)

[worin

$X_a$, $X_b$, $X_c$, $X_d$, $X_e$ und $X_f$ jeweils unabhängig eine Alkylengruppe mit 1 bis 4 Kohlenstoffatomen darstellen,

$R_a$, $R_{b'}$ $R_c$, $R_{cc}$, $R_d$, $R_{dd}$, $R_e$, $R_{ee}$, $R_f$ und $R_{ff}$ jeweils unabhängig ausgewählt sind aus einem Halogenatom, einer Alkylgruppe mit 1 bis 20 Kohlenstoffatomen, einer Alkoxygruppe mit 1 bis 20 Kohlenstoffatomen, einer Cycloalkylgruppe mit 5 bis 20 Kohlenstoffatomen, einer Cycloalkoxygruppe mit 5 bis 20 Kohlenstoffatomen, einer Arylgruppe mit 6 bis 20 Kohlenstoffatomen, einer Heteroarylgruppe mit 3 bis 20 Kohlenstoffatomen, die ein oder mehrere heterocyclische Atome enthält, die ausgewählt sind aus O, N und S, einer Aryloxygruppe mit 6 bis 20 Kohlenstoffatomen und $-C{\equiv}C-R_i$,

$R_i$ eine Arylgruppe mit 6 bis 20 Kohlenstoffatomen oder eine Heteroarylgruppe mit 3 bis 20 Kohlenstoffatomen darstellt, die ein oder mehrere heterocyclische Atome enthält, die ausgewählt sind aus O, N und S,

a, b, c, d, e und f jeweils unabhängig eine ganze Zahl von 0 bis 10 darstellen,

h, i, j, j', k, k', m, m', n und n' jeweils unabhängig eine ganze Zahl von 0 bis 4 darstellen, und

jedes $R_g$ unabhängig ein Wasserstoffatom oder eine Alkylgruppe mit 1 bis 3 Kohlenstoffatomen darstellt]

behandelt wird, um eine Reaktionslösung zu erhalten, die ein erstes Lösungsmittel und eine Mischung aus Dihydroxyverbindungen enthält, enthaltend eine erste Dihydroxyverbindung, die ausgewählt ist aus der Gruppe bestehend aus den unten angegebenen allgemeinen Formeln (1') bis (4'):

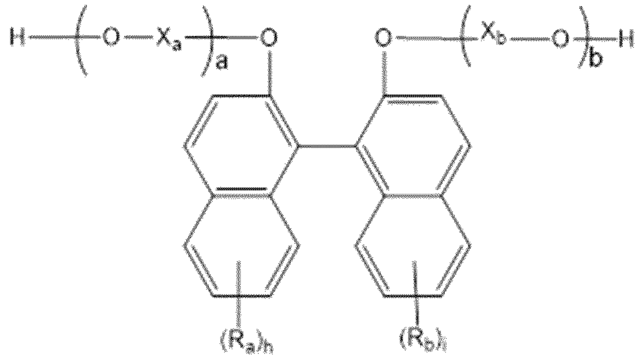

(1')

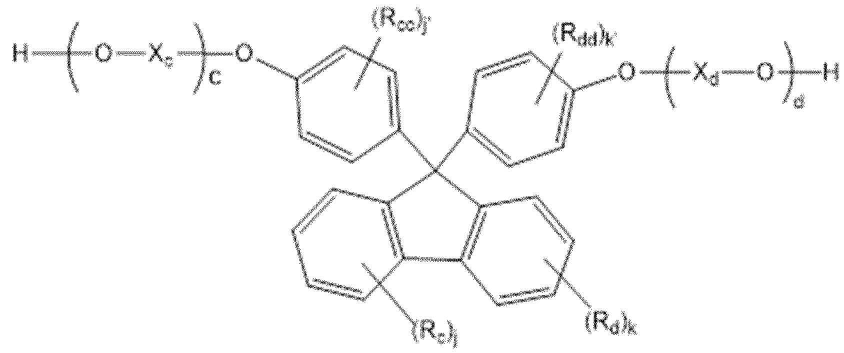

(2')

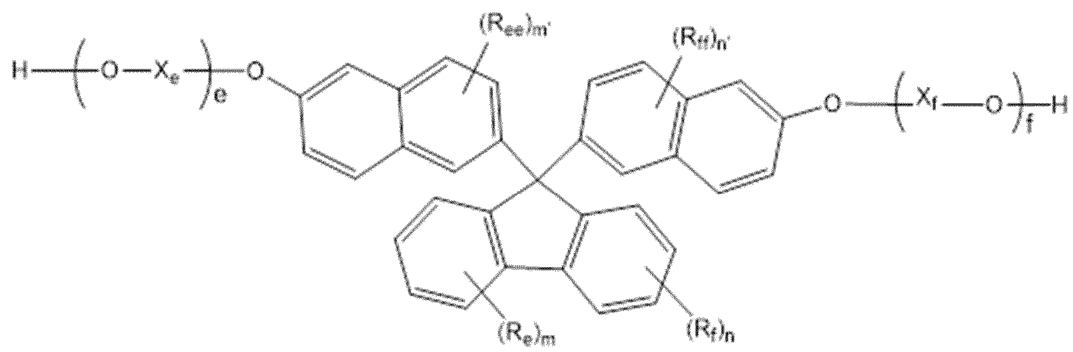

(3')

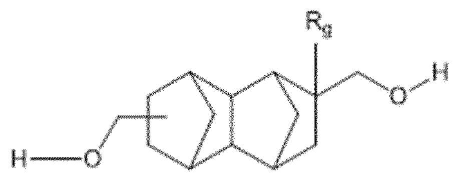

(4')

[worin jedes Symbol dasselbe wie in den oben angegebenen allgemeinen Formeln (1) bis (4) darstellt]
und mindestens eine weitere Dihydroxyverbindung, die ausgewählt ist aus der Gruppe bestehend aus den allgemeinen Formeln (1') bis (4'); und
einen Schritt (b1), in dem die erste Dihydroxyverbindung aus einer Kristallisationslösung kristallisiert wird, die erhalten wird durch Zugabe eines zweiten Lösungsmittels zu der Reaktionslösung,
wobei die Differenz zwischen der Löslichkeit der ersten Dihydroxyverbindung in dem zweiten Lösungsmittel bei 25 °C und der Löslichkeit der anderen Dihydroxyverbindung in dem zweiten Lösungsmittel bei 25 °C (Löslichkeit der anderen Dihydroxyverbindung minus Löslichkeit der ersten Dihydroxyverbindung) größer oder gleich 0,1 g/(10 ml) beträgt.

**2.** Verfahren gemäß Anspruch 1, wobei die Kristallisationslösung ferner einen Impfkristall enthält.

**3.** Verfahren gemäß Anspruch 1 oder 2, wobei der Gehalt des ersten Lösungsmittels in der Kristallisationslösung 0,1 bis 10 g/g, bezogen auf die Gesamtmenge der Mischung der Dihydroxyverbindungen, beträgt.

4. Verfahren gemäß mindestens einem der Ansprüche 1 bis 3, wobei der Gehalt des zweiten Lösungsmittels in der Kristallisationslösung 0,3 bis 3 g/g, bezogen auf die Gesamtmenge der Mischung der Dihydroxyverbindungen, beträgt.

5. Verfahren gemäß mindestens einem der Ansprüche 1 bis 4, wobei der Gesamtgehalt des ersten Lösungsmittels und des zweiten Lösungsmittels in der Kristallisationslösung 1 bis 10 g/g, bezogen auf die Gesamtmenge der Mischung der Dihydroxyverbindungen, beträgt.

6. Verfahren gemäß mindestens einem der Ansprüche 1 bis 5, wobei das Verhältnis des Gehalts (g/g) des ersten Lösungsmittels zum Gehalt (g/g) des zweiten Lösungsmittels in der Kristallisationslösung (erstes Lösungsmittel/-zweites Lösungsmittel) zwischen 0,8 und 10 liegt.

7. Verfahren zur Herstellung einer Dihydroxyverbindung aus einer Abfallharzzusammensetzung, wobei das Verfahren umfasst:

einen Schritt (a2), in dem eine alkalische Lösung, die ein erstes Lösungsmittel, Wasser und eine Abfallharzzusammensetzung enthält, die ein Harz mit mindestens zwei Struktureinheiten enthält, die ausgewählt sind aus der Gruppe bestehend aus den unten angegebenen allgemeinen Formeln (1) bis (4):

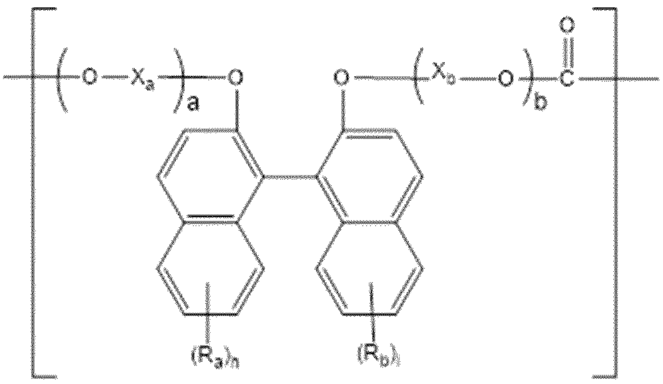

(1)

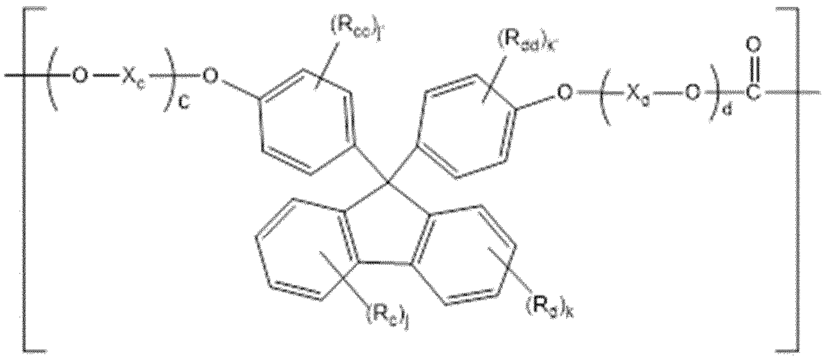

(2)

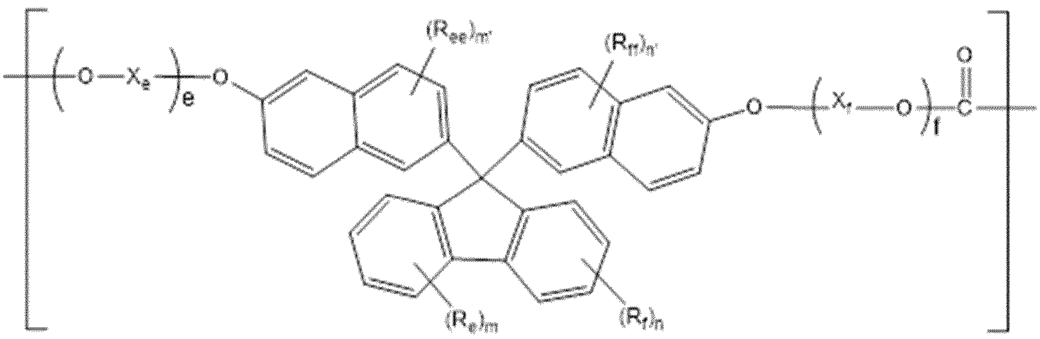

(3)

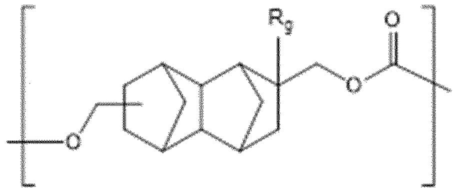

(4)

[worin

$X_a$, $X_b$, $X_c$, $X_d$, $X_e$ und $X_f$ jeweils unabhängig eine Alkylengruppe mit 1 bis 4 Kohlenstoffatomen darstellen,

$R_a$, $R_b$, $R_c$, $R_{cc}$, $R_d$, $R_{dd}$, $R_e$, $R_{ee}$, $R_f$ und $R_{ff}$ jeweils unabhängig ausgewählt sind aus einem Halogenatom, einer Alkylgruppe mit 1 bis 20 Kohlenstoffatomen, einer Alkoxygruppe mit 1 bis 20 Kohlenstoffatomen, einer Cycloalkylgruppe mit 5 bis 20 Kohlenstoffatomen, einer Cycloalkoxygruppe mit 5 bis 20 Kohlenstoffatomen, einer Arylgruppe mit 6 bis 20 Kohlenstoffatomen, einer Heteroarylgruppe mit 3 bis 20 Kohlenstoffatomen, die ein oder mehrere heterocyclische Atome enthält, die ausgewählt sind aus O, N und S, einer Aryloxygruppe mit 6 bis 20 Kohlenstoffatomen und $-C{\equiv}C-R_i$,

$R_i$ eine Arylgruppe mit 6 bis 20 Kohlenstoffatomen oder eine Heteroarylgruppe mit 3 bis 20 Kohlenstoffatomen darstellt, die ein oder mehrere heterocyclische Atome enthält, die ausgewählt sind aus O, N und S,

a, b, c, d, e und f jeweils unabhängig eine ganze Zahl von 0 bis 10 darstellen,

h, i, j, j', k, k', m, m', n und n' jeweils unabhängig eine ganze Zahl von 0 bis 4 darstellen, und

jedes $R_g$ unabhängig ein Wasserstoffatom oder eine Alkylgruppe mit 1 bis 3 Kohlenstoffatomen darstellt]

behandelt wird, um eine Reaktionslösung zu erhalten, die ein erstes Lösungsmittel und eine Mischung aus Dihydroxyverbindungen enthält, enthaltend eine erste Dihydroxyverbindung, die ausgewählt ist aus der Gruppe bestehend aus den unten angegebenen allgemeinen Formeln (1') bis (4'):

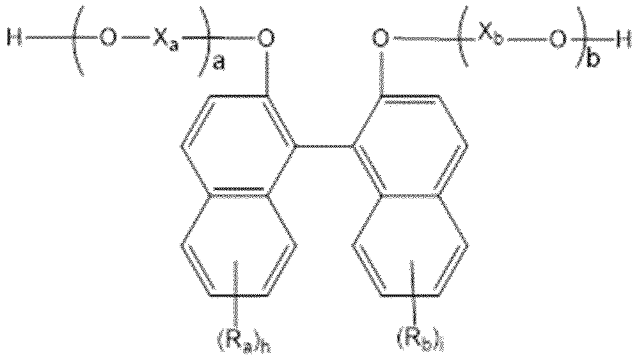

(1')

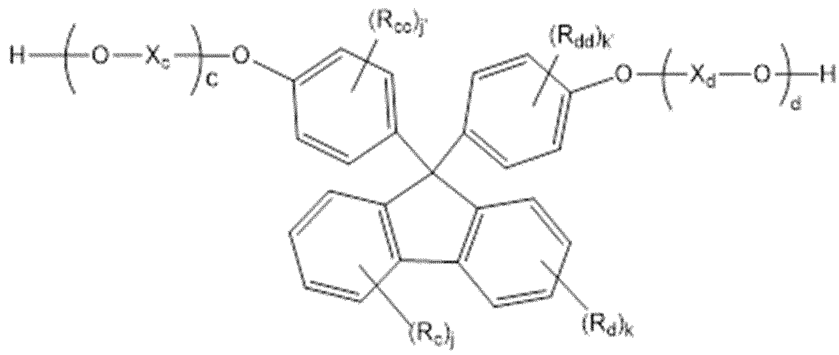

(2')

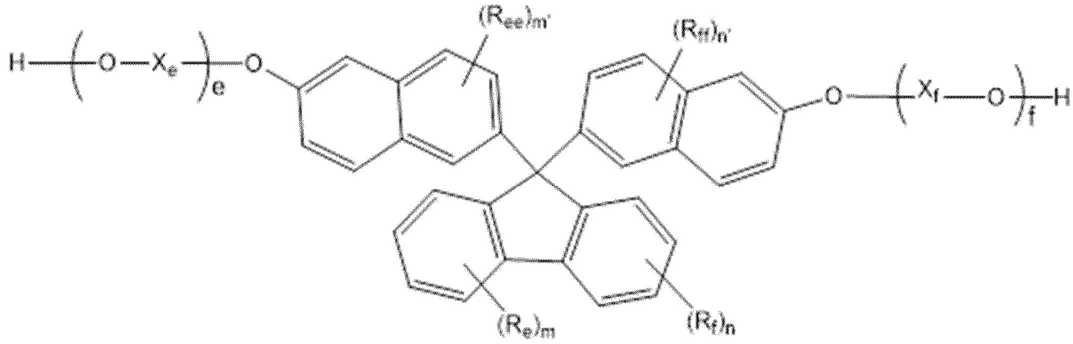

(3')

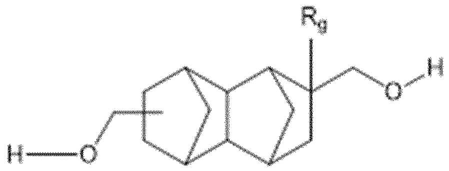

(4')

[worin jedes Symbol dasselbe wie in den oben angegebenen allgemeinen Formeln (1) bis (4) darstellt] und mindestens eine weitere Dihydroxyverbindung, die ausgewählt ist aus der Gruppe bestehend aus den allgemeinen Formeln (1') bis (4'); und
einen Schritt (b2), in dem mindestens die erste Dihydroxyverbindung aus der Reaktionslösung kristallisiert wird, wobei der Schritt (b2) das Erwärmen einer Reaktionslösung auf 80 °C oder höher umfasst.

**8.** Verfahren gemäß mindestens einem der Ansprüche 1 bis 7, wobei die Lösung der Abfallharzzusammensetzung ein Verunreinigungsharz enthält, das mindestens eine Struktureinheit aufweist, die ausgewählt ist aus der Gruppe bestehend aus den unten angegebenen allgemeinen Formeln (6) bis (8):

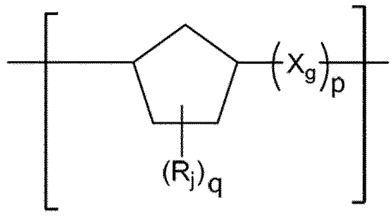

(6)

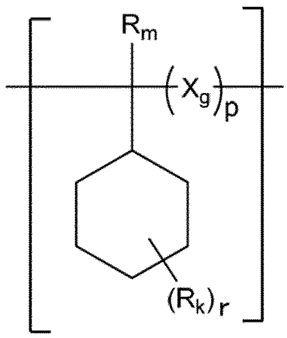

(7)

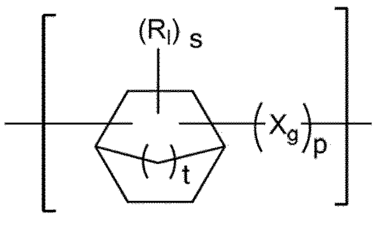

(8)

[worin

jedes $X_g$ unabhängig eine Alkylengruppe mit 1 bis 10 Kohlenstoffatomen darstellt;
$R_j$, $R_k$ und $R_l$ jeweils unabhängig ausgewählt sind aus einem Halogenatom, einer substituierten oder unsubstituierten Alkylgruppe mit 1 bis 20 Kohlenstoffatomen, einer substituierten oder unsubstituierten Alkoxygruppe mit 1 bis 20 Kohlenstoffatomen, einer substituierten oder unsubstituierten Cycloalkylgruppe mit 5 bis 20 Kohlenstoffatomen, einer substituierten oder unsubstituierten Cycloalkoxygruppe mit 5 bis 20 Kohlenstoffatomen, einer substituierten oder unsubstituierten Arylgruppe mit 6 bis 20 Kohlenstoffatomen, einer substituierten oder unsubstituierten Heteroarylgruppe mit 3 bis 20 Kohlenstoffatomen, die ein oder mehrere heterocyclische Atome enthält, die ausgewählt sind aus O, N und S, einer substituierten oder unsubstituierten Aryloxygruppe mit 6 bis 20 Kohlenstoffatomen und $-C\equiv C-R_i$,
$R_i$ eine substituierte oder unsubstituierte Arylgruppe mit 6 bis 20 Kohlenstoffatomen oder eine Heteroarylgruppe mit 3 bis 20 Kohlenstoffatomen darstellt, die ein oder mehrere heterocyclische Atome enthält, die ausgewählt sind aus O, N und S,
jedes p unabhängig eine ganze Zahl von 0 oder 1 darstellt,
q, r und s jeweils unabhängig eine ganze Zahl von 0 bis 10 darstellen,
t eine ganze Zahl von 1 bis 3 darstellt,
wobei, wenn q 2 oder mehr ist und zwei $R_j$ an benachbarten Kohlenstoffatomen vorhanden sind, die beiden $R_j$ miteinander verbunden sein können, um eine Ringstruktur zu bilden,
wenn r 2 oder mehr ist und zwei $R_k$ an benachbarten Kohlenstoffatomen vorhanden sind, die beiden $R_k$ miteinander verbunden sein können, um eine Ringstruktur zu bilden,
wenn s 2 oder mehr ist und zwei $R_l$ an benachbarten Kohlenstoffatomen vorhanden sind, die beiden $R_l$ miteinander verbunden sein können, um eine Ringstruktur zu bilden, und
$R_m$ ein Wasserstoffatom oder eine Alkylgruppe mit 1 bis 3 Kohlenstoffatomen darstellt].

**9.** Verfahren zur Herstellung eines recycelten Harzes, umfassend das Polymerisieren einer Dihydroxyverbindung, die gemäß mindestens einem der Verfahren gemäß den Ansprüchen 1 bis 8 hergestellt wurde.

## Revendications

**1.** Procédé de préparation d'un composé dihydroxylé à partir d'une composition de résine de rebut, le procédé comprenant :

une étape (a1) dans laquelle une solution alcaline contenant un premier solvant, de l'eau et une composition de résine de rebut contenant une résine présentant au moins deux motifs constitutifs sélectionnés dans le groupe consistant en les formules générales (1) à (4) présentées ci-dessous :

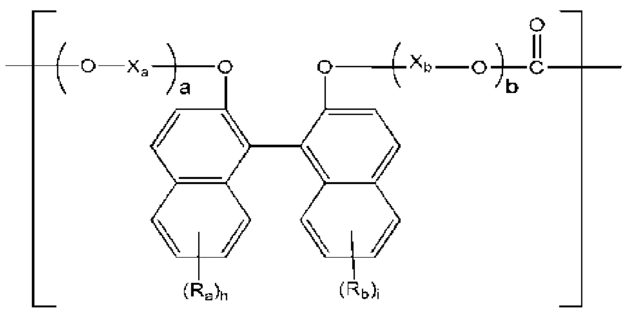

(1)

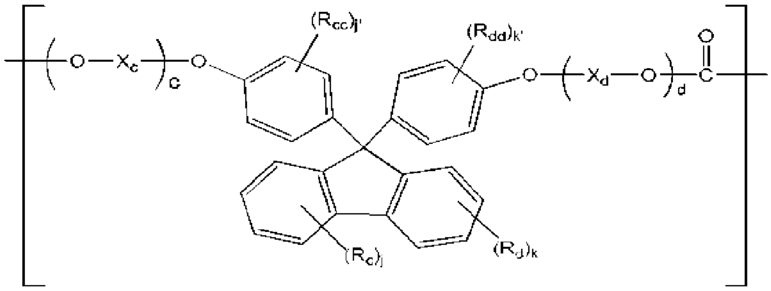

(2)

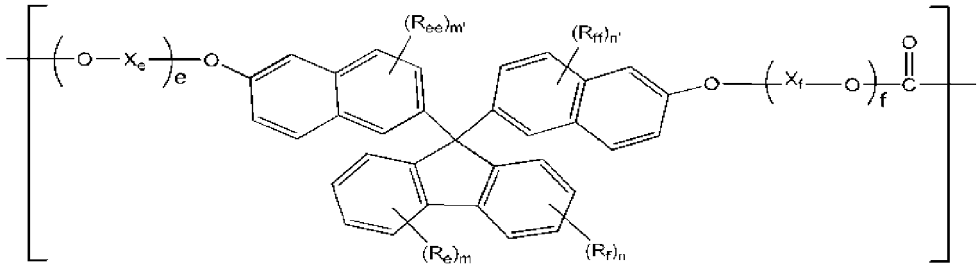

(3)

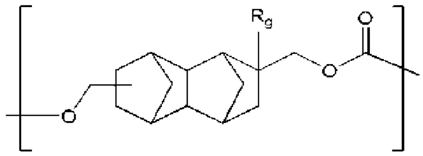

(4)

[dans lequel
$X_a$, $X_b$, $X_c$, $X_d$, $X_e$, et $X_f$ représentent chacun indépendamment un groupe alkylène présentant 1 à 4 atomes de carbone,
$R_a$, $R_b$, $R_c$, $R_{cc}$, $R_d$, $R_{dd}$, $R_e$, $R_{ee}$, $R_f$ et $R_{ff}$ sont sélectionnés chacun indépendamment parmi un atome d'halogène, un groupe alkyle présentant 1 à 20 atomes de carbone, un groupe alcoxy présentant 1 à 20 atomes de carbone, un groupe cycloalkyle présentant 5 à 20 atomes de carbone, un groupe cycloalcoxy présentant 5 à 20 atomes de carbone, un groupe aryle présentant 6 à 20 atomes de carbone, un groupe hétéroaryle présentant 3 à 20 atomes de carbone et contenant un ou plusieurs atomes hétérocycliques sélectionnés parmi O, N, et S, un groupe aryloxy présentant 6 à 20 atomes de carbone, et $-C{\equiv}C-R_i$,
$R_i$ représente un groupe aryle présentant 6 à 20 atomes de carbone ou un groupe hétéroaryle présentant 3 à 20 atomes de carbone et contenant un ou plusieurs atomes hétérocycliques sélectionnés parmi O, N et S,
a, b, c, d, e et f représentent chacun indépendamment un nombre entier de 0 à 10, h, i, j, j', k, k', m, m', n, et n' représentent chacun indépendamment un nombre entier de 0 à 4, et
chaque $R_g$ représente indépendamment un atome d'hydrogène ou un groupe alkyle présentant 1 à 3 atomes de carbone]
est traitée pour donner une solution réactionnelle contenant un premier solvant et un mélange de composés dihydroxylés contenant un premier composé dihydroxylé sélectionné dans le groupe consistant en les formules générales (1') à (4') présentées ci-dessous :

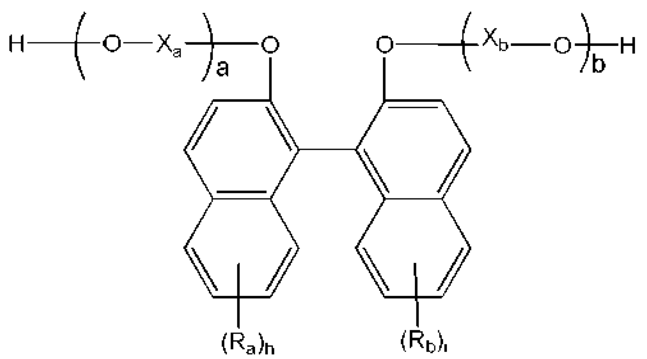

(1')

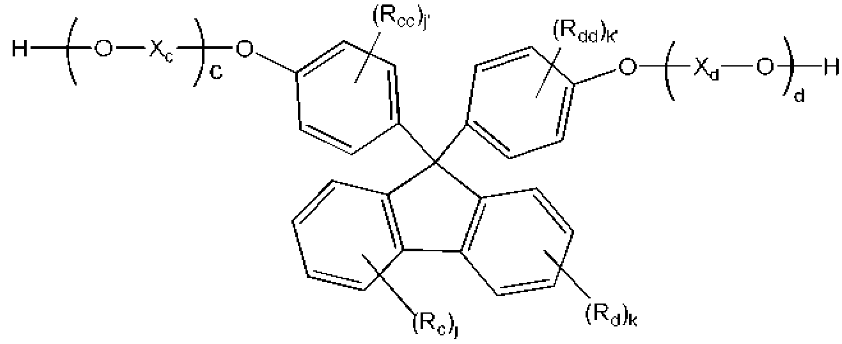

(2')

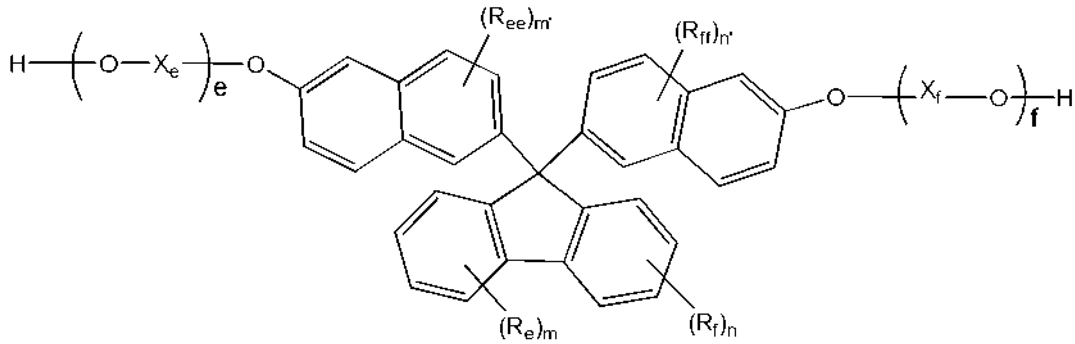

(3')

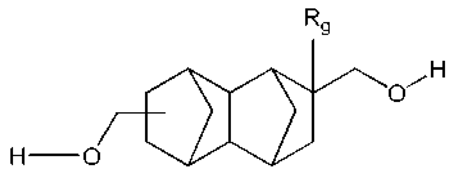

(4')

[dans lequel chaque symbole représente la même chose que dans les formules générales (1) à (4) présentées ci-dessus]

et au moins un autre composé dihydroxylé sélectionné dans le groupe consistant en les formules générales (1') à (4') ; et

une étape (b1) dans laquelle le premier composé dihydroxylé est cristallisé à partir d'une solution de cristallisation obtenue par ajout d'un second solvant à la solution réactionnelle,

dans lequel une différence entre la solubilité du premier composé dihydroxylé dans le second solvant à 25 °C et la solubilité de l'autre composé dihydroxylé dans le second solvant à 25 °C (solubilité de l'autre composé dihydroxylé moins solubilité du premier composé dihydroxy) est supérieure ou égale à 0,1 g/(10 ml).

**2.** Procédé selon la revendication 1, dans lequel la solution de cristallisation contient en outre un germe cristallin.

**3.** Procédé selon la revendication 1 ou la revendication 2, dans lequel une teneur du premier solvant dans la solution de cristallisation est de 0,1 à 10 g/g par rapport à une quantité totale du mélange des composés dihydroxylés.

**4.** Procédé selon l'une quelconque des revendications 1 à 3, dans lequel une teneur du second solvant dans la solution de cristallisation est de 0,3 à 3 g/g par rapport à une quantité totale du mélange des composés dihydroxylés.

**5.** Procédé selon l'une quelconque des revendications 1 à 4, dans lequel une teneur totale du premier solvant et du second solvant dans la solution de cristallisation est de 1 à 10 g/g par rapport à une quantité totale du mélange des composés dihydroxylés.

**6.** Procédé selon l'une quelconque des revendications 1 à 5, dans lequel un rapport d'une teneur (g/g) du premier solvant sur une teneur (g/g) du second solvant dans la solution de cristallisation (premier solvant/second solvant) est de 0,8 à 10.

**7.** Procédé de préparation d'un composé dihydroxylé à partir d'une composition de résine de rebut, le procédé comprenant :

une étape (a2) dans laquelle une solution alcaline contenant un premier solvant, de l'eau et une composition de résine de rebut contenant une résine présentant au moins deux motifs constitutifs sélectionnés dans le groupe consistant en les formules générales (1) à (4) présentées ci-dessous :

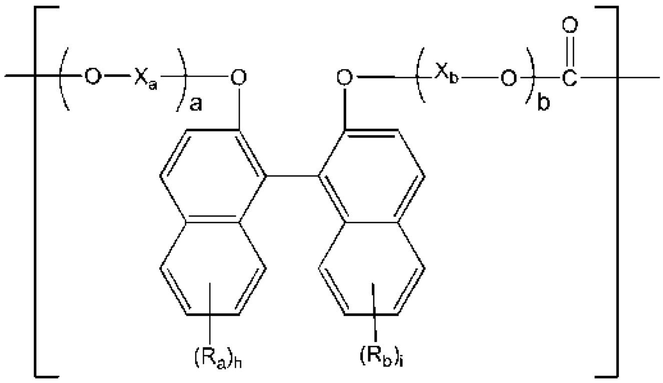

(1)

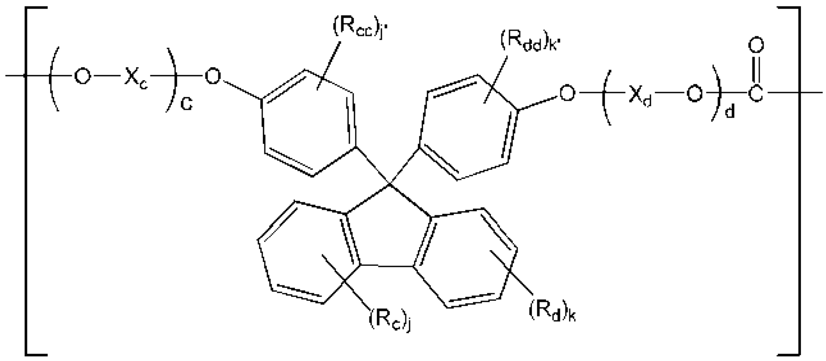

(2)

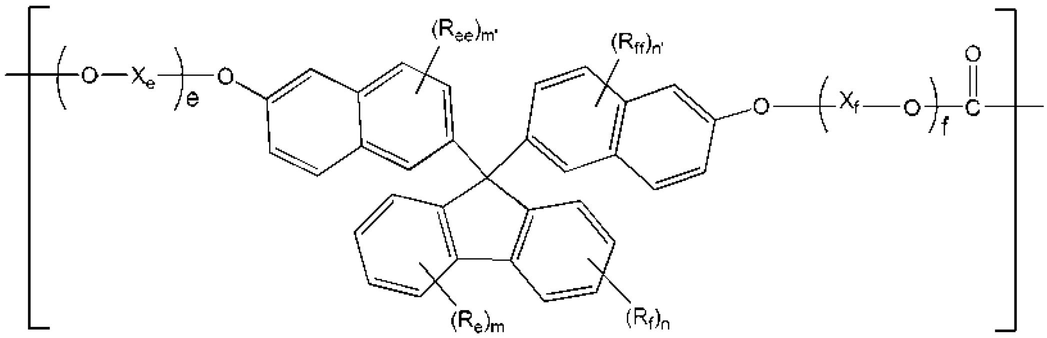

(3)

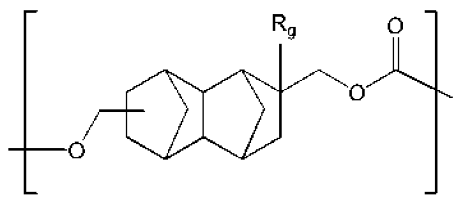

(4)

[dans lequel

$X_a$, $X_b$, $X_c$, Xd, $X_e$, et $X_f$ représentent chacun indépendamment un groupe alkylène présentant 1 à 4 atomes de carbone,

$R_a$, $R_b$, $R_c$, $R_{cc}$, $R_d$, $R_{dd}$, $R_e$, $R_{ee}$, $R_f$, et $R_{ff}$ sont sélectionnés chacun indépendamment parmi un atome d'halogène, un groupe alkyle présentant 1 à 20 atomes de carbone, un groupe alcoxy présentant 1 à 20 atomes de carbone, un groupe cycloalkyle présentant 5 à 20 atomes de carbone, un groupe cycloalcoxy présentant 5 à 20 atomes de carbone, un groupe aryle présentant 6 à 20 atomes de carbone, un groupe hétéroaryle présentant 3 à 20 atomes de carbone et contenant un ou plusieurs atomes hétérocycliques sélectionnés parmi O, N, et S, un

groupe aryloxy présentant 6 à 20 atomes de carbone, et -C≡C-$R_i$,

$R_i$ représente un groupe aryle présentant 6 à 20 atomes de carbone ou un groupe hétéroaryle présentant 3 à 20 atomes de carbone et contenant un ou plusieurs atomes hétérocycliques sélectionnés parmi O, N et S,

a, b, c, d, e et f représentent chacun indépendamment un nombre entier de 0 à 10, h, i, j, j', k, k', m, m', n, et n' représentent chacun indépendamment un nombre entier de 0 à 4, et

chaque $R_g$ représente indépendamment un atome d'hydrogène ou un groupe alkyle présentant 1 à 3 atomes de carbone]

est traitée pour donner une solution réactionnelle contenant un premier solvant et un mélange de composés dihydroxylés contenant un premier composé dihydroxylé sélectionné dans le groupe consistant en les formules générales (1') à (4') présentées ci-dessous :

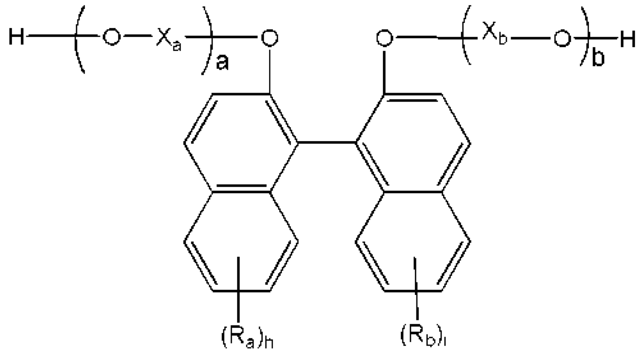

(1')

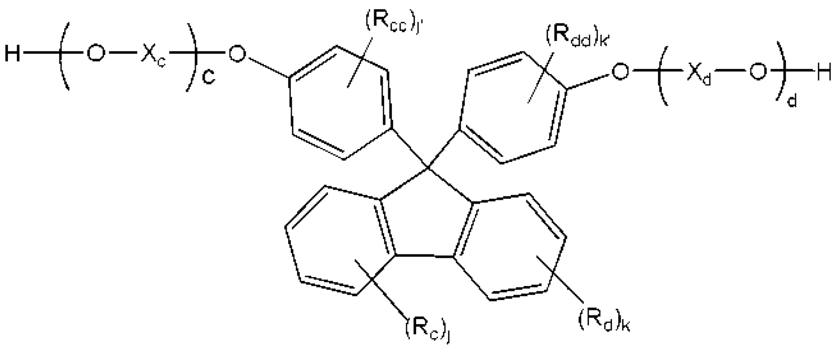

(2')

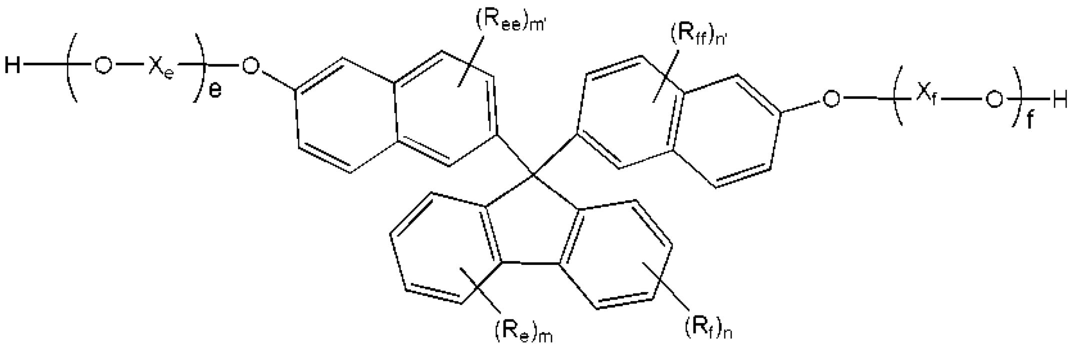

(3')

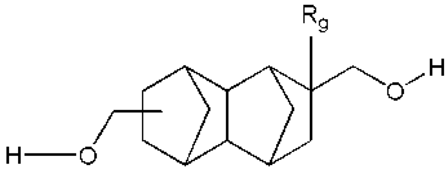

(4')

[dans lequel chaque symbole représente la même chose que dans les formules générales (1) à (4) présentées ci-dessus]

et au moins un autre composé dihydroxylé sélectionné dans le groupe consistant en les formules générales (1) à

(4') ; et

une étape (b2) dans laquelle au moins le premier composé dihydroxylé est cristallisé à partir de la solution réactionnelle,

dans lequel l'étape (b2) comprend le chauffage d'une solution réactionnelle à 80 °C ou plus.

**8.** Procédé selon l'une quelconque des revendications 1 à 7, dans lequel la solution de la composition de résine de rebut contient une résine d'impureté présentant au moins un motif constitutif sélectionné dans le groupe consistant en les formules générales (6) à (8) présentées ci-dessous :

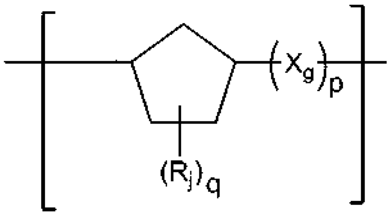
(6)

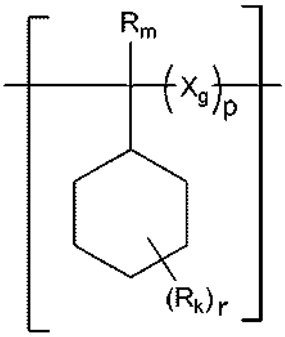
(7)

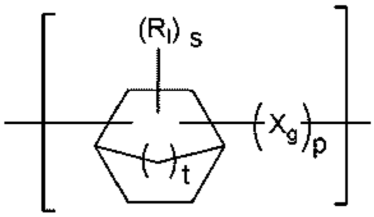
(8)

[dans lequel

chaque $X_g$ représente indépendamment un groupe alkylène présentant 1 à 10 atomes de carbone ;

$R_j$, $R_k$, et $R_l$ sont chacun sélectionnés indépendamment parmi un atome d'halogène, un groupe alkyle substitué ou non substitué présentant 1 à 20 atomes de carbone, un groupe alcoxy substitué ou non substitué présentant 1 à 20 atomes de carbone, un groupe cycloalkyle substitué ou non substitué présentant 5 à 20 atomes de carbone, un groupe cycloalcoxy substitué ou non substitué présentant 5 à 20 atomes de carbone, un groupe aryle substitué ou non substitué présentant 6 à 20 atomes de carbone, un groupe hétéroaryle substitué ou non substitué présentant 3 à 20 atomes de carbone et contenant un ou plusieurs atomes hétérocycliques sélectionnés parmi O, N et S, un groupe aryloxy substitué ou non substitué présentant 6 à 20 atomes de carbone, et $-C{\equiv}C-R_i$,

$R_i$ représente un groupe aryle substitué ou non substitué présentant 6 à 20 atomes de carbone ou un groupe hétéroaryle présentant 3 à 20 atomes de carbone et contenant un ou plusieurs atomes hétérocycliques sélectionnés parmi O, N et S,

chaque p représente indépendamment un nombre entier égal à 0 ou 1,

q, r et s représentent chacun indépendamment un nombre entier de 0 à 10,

t représente un nombre entier de 1 à 3,

dans lequel lorsque q est 2 ou plus et que deux $R_j$ sont présents sur des atomes de carbone adjacents, les deux $R_j$ peuvent être liés ensemble pour former une structure cyclique,

lorsque r est 2 ou plus et que deux $R_k$ sont présents sur des atomes de carbone adjacents, les deux $R_k$ peuvent être liés ensemble pour former une structure cyclique,

lorsque s est 2 ou plus et que deux $R_l$ sont présents sur des atomes de carbone adjacents, les deux $R_l$ peuvent être liés ensemble pour former une structure cyclique,
$R_m$ représente un atome d'hydrogène ou un groupe alkyle présentant 1 à 3 atomes de carbone].

**9.** Procédé de préparation d'une résine recyclée, comprenant la polymérisation d'un composé dihydroxylé préparé par le procédé selon l'une quelconque des revendications 1 à 8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011131507 A